# EUROPEAN PATENT SPECIFICATION

(11) **EP 2 968 363 B1**
(45) Date of publication and mention of the grant of the patent: **19.08.2020**
(21) Application number: 14764607.9
(22) Date of filing: 17.03.2014
(51) Int. Cl.: A61K 31/568, A61K 9/48, A61P 5/26

(54) **LIPOBALANCED LONG CHAIN TESTOSTERONE ESTERS FOR ORAL DELIVERY**
LIPOBALANCIERTE LANGKETTIGE TESTOSTERONESTER ZUR ORALEN VERABREICHUNG
ESTERS DE TESTOSTÉRONE À LONGUE CHAÎNE LIPOÉQUILIBRÉS POUR UNE ADMINISTRATION ORALE

(30) Priority: 15.03.2013 US 201313843403
(43) Date of publication of application: 20.01.2016
(73) Proprietor: Lipocine Inc., Salt Lake City, UT 84108 (US)
(72) Inventor: NACHAEGARI, Satish Kumar, Holladay, UT 84117 (US); GILIYAR, Chandrashekar, Salt Lake City, Utah 84124 (US); PATEL, Raj, Salt Lake City, Utah 84124 (US); NACHIAPPAN, Chidambaram, Sandy, Utah 84070 (US); VENKATESHWARAN, Srinivansan, Salt Lake City, Utah 84108 (US); PATEL, Mahesh V., Salt Lake City, Utah 84124 (US)
(74) Representative: Vossius & Partner Patentanwälte Rechtsanwälte mbB
(86) International application number: PCT/US2014/030604
(87) International publication number: WO 2014/145781

(56) References cited:
- US-A- 4 147 783
- US-A1- 2003 022 875
- US-A1- 2005 101 517
- US-A1- 2011 251 167
- US-A1- 2012 244 215
- US-B2- 7 138 389
- US-B2- 7 718 640
- US-B2- 8 241 664
- EMILY PFEIL ET AL: "Current and future testosterone delivery systems for treatment of the hypogonadal male", EXPERT OPINION ON DRUG DELIVERY, vol. 5, no. 4, April 2008 (2008-04), pages 471-481, XP055284481, GB ISSN: 1742-5247, DOI: 10.1517/17425247.5.4.471
- MARIE-LAURE LEICHTNAM ET AL: "Testosterone Hormone Replacement Therapy: State-of-the-Art and Emerging Technologies", PHARMACEUTICAL RESEARCH, KLUWER ACADEMIC PUBLISHERS-PLENUM PUBLISHERS, NL, vol. 23, no. 6, 9 June 2006 (2006-06-09), pages 1117-1132, XP019405115, ISSN: 1573-904X, DOI: 10.1007/S11095-006-0072-5

## Description

### FIELD OF THE INVENTION

The present invention relates to pharmaceutical compositions and dosage forms containing select testosterone esters for use in the treatment of a hypogonadal male, as further defined in the claims. Accordingly, this invention involves the fields of chemistry, pharmaceutical sciences, medicine and other health sciences.

### BACKGROUND OF THE INVENTION

An average human male produces about four to seven milligram of testosterone (T) per day in a circadian pattern, with maximal serum levels attained in the early morning and minimal levels in the evening. It is generally recognized that in a normal adult man of age 17 to 54 years, the serum total T is between about 300 ng/dL to about 1100 ng/dL and this range is referred to as the eugonadal range. Male hypogonadism (serum total T < 300 ng/dL) is a serious condition affecting mostly aging men. The common reasons for hypogonadism in men could be physiological abnormality involving among other factors, improper functioning or growth of the gonads and/or the pituitaryhypothalamus regulatory systems, and/or aging. Restoration of serum T levels to the eugonadal range typically corrects many of the clinical abnormalities associated with hypogonadism or low testosterone levels.

Currently, oral modified testosterones, in the form of a methyl analogue of T, and as an undecanoate ester, testosterone undecanoate (TU), are available for oral administration for patients in need of testosterone therapy. However, liver damage including cholestasis, peliosis hepatitis, nodular regenerative hyperplasia, and primary hepatic tumors are reported for instance with use of methyl testosterone. Testosterone ester with low lipophilicity (clog P <10) such as T ester of medium chain fatty acid esters are not particularly effective due to the inability of these esters to deliver longer lasting testosterone in the blood resulting in an inconvenient high dosing frequency regimen. Therapies involving testosterone undecanoate upon single daily dose oral administration appear to offer inadequate benefits due to the sub-optimal, short acting, serum T profiles. Specifically, such testosterone undecanoate administrations serum T levels often remain in the hypogonadal range (<300ng/dL) for a large proportion of the dosing period (usually >7 hours in a 24-hour period) and in a larger percentage of patients (i.e. > 60%) in a group of patients receiving such therapy. Moreover, impractical dosage regimen such as higher T equivalent daily dose, frequent administration in a day, and more number of dosage units per administration present patient-compliance issues negatively affecting the effectiveness of such therapies involving oral testosterone undecanoate.

### SUMMARY OF THE INVENTION

The present invention relates to the following embodiments:
(1) An oral pharmaceutical composition for use in the treatment of a condition associated with testosterone deficiency in a human subject, the composition comprising:
   a testosterone ester having a structure: 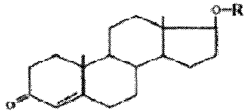
   wherein R is -C₁₃H₂₅O or -C₁₄H₂₇O, and one or both esters can be present in the composition; and
   a pharmaceutically acceptable carrier,
   wherein the composition is formulated as an oral dosage form,
   wherein the treatment comprises administering to the human subject the oral dosage form to provide a daily testosterone ester dose of about 420 mg to about 1250 mg based on single unit or multiple unit dosing, and
   wherein the human subject is a hypogonadal male.
(2) The oral pharmaceutical composition for use according to embodiment (1) wherein, upon single administration to a group of hypogonadal males, the composition is capable of providing a mean serum testosterone C_{avg 12-24h} that is within about 35% to about 70% of the mean serum testosterone C_{avg 0-24h}.
(3) The oral pharmaceutical composition for use according to embodiment (1) or (2), wherein the dosage form is formulated for once-a-day administration.
(4) The oral pharmaceutical composition for use according embodiment (1) or (2), which is based on multiple unit dosing and comprises administering the entire required dose of the testosterone ester per administration time in the form of two or more unit dosage forms.
(5) The oral pharmaceutical composition for use of embodiment (1) or (2), wherein R is -C₁₃H₂₅O and when a daily dose of 420 mg to 850 mg testosterone ester is administered continuous once-a-day to each subject in a group of hypogonadal males for a period of at least 84 days, the administration is such that at least 75% of the hypogonadal males in the group have a serum testosterone C_{avg} of about 300ng/dL to about 1100 ng/dL, and at least one of the following:
   a serum testosterone Cₘₐₓ of less than 1500 ng/dL in at least 85% of the subjects in the group;
   a serum testosterone Cₘₐₓ of about 1800 ng/dL to about 2500 ng/dL in 10% or less of the subjects in the group; and
   a serum testosterone Cₘₐₓ greater than 2500 ng/dL in about 5% or less of the subjects in the group.
(6) The oral pharmaceutical composition for use of embodiment (1) or (2), wherein R is -C₁₄H₂₇O and when a daily dose of 525 mg to 1250 mg testosterone ester is administered continuous once-a-day to each subject in a group of hypogonadal males for a period of at least 84 days, the administration is such that at least 75% of the hypogonadal males in the group have a serum testosterone C_{avg} of about 300ng/dL to about 1100 ng/dL, and at least one of the following:
   a serum testosterone Cₘₐₓ of less than 1500 ng/dL in at least 85% of the subjects in the group;
   a serum testosterone Cₘₐₓ of about 1800 ng/dL to about 2500 ng/dL in 10% or less of the subjects in the group; and
   a serum testosterone Cₘₐₓ greater than 2500 ng/dL in about 5% or less of the subjects in the group.
(7) The oral pharmaceutical composition for use of embodiment (1) or (2), wherein the pharmaceutically acceptable carrier includes a lipophilic additive which is a lipophilic surfactant.

In the following, embodiments according to the present invention (as defined in the claims) as well as reference embodiments will be described. Embodiments disclosed herein which do not fall within the scope of the claims do not form part of the invention.

The present disclosure is drawn to oral pharmaceutical compositions and dosage forms containing select testosterone esters. In one embodiment, an oral pharmaceutical composition for administration to human subjects in need of testosterone is provided.
The composition comprises a testosterone ester and a pharmaceutically acceptable carrier. The testosterone ester can have the structure 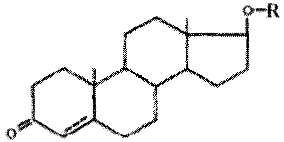 wherein R is -C₁₃H₂₅O or -C₁₄H₂₇O. One or both of the esters can be present in the pharmaceutical composition. The composition is formulated such that upon single dose administration to a group of human subjects, the composition provides a mean serum testosterone C_{avg t12-t24} that is within about 35% to about 70% of the mean serum testosterone C_{avg t0-t24}.

In another embodiment, an oral pharmaceutical composition for administration to subjects in need of testosterone therapy is provided that includes a testosterone ester and a pharmaceutically acceptable carrier. The testosterone ester can have the structure: 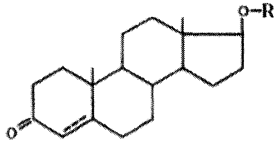 wherein, wherein R is -C₁₃H₂₅O or -C₁₄H₂₇O. Further, the composition can include one or both of the T13 or T14 testosterone esters. Further, the composition can be formulated such that upon administration of a daily dose of about 420 mg to about 1250 mg of the testosterone ester to each subject in a group of at least 12 hypogonadal males for a period of at least 84 days, 50% or less of the subjects in the group have a serum testosterone concentration that falls below 300 ng/dL for more than 7 hours per day at steady state.

In an additional embodiment, a capsule dosage form for oral administration of a testosterone ester is provided. The capsule dosage form can include about 100 mg to about 400 mg of at least one testosterone ester and a lipophilic additive. The testosterone ester can have the structure: 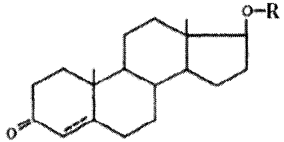 wherein R is -C₁₃H₂₅O or -C₁₄H₂₇O and one or both esters can be present in the dosage form.
The capsule dosage form can be formulated such that the testosterone ester is not fully dissolved at about 20°C in the at least one lipophilic additive.

In still a further embodiment, the above-described oral pharmaceutical composition or dosage form for use in a method of treating a human subject in need of testosterone therapy is provided. The method can include the steps of administering an oral pharmaceutical composition or capsule dosage form disclosed herein.

### BRIEF DESCRIPTION OF THE DRAWINGS

FIG. 1 is a plot of the solubility of several testosterone esters in oleic acid.
FIG. 2 shows a plot of the solubility of several testosterone esters in castor oil.
FIG. 3 shows a plot of solubility data of various testosterone esters in mono-, diglyceride (glyceryl mono-, di- linoleate).
FIG. 4 shows a plot of Cₘₐₓ and Tₘₐₓ normalized post-Tₘₐₓ serum testosterone concentrations (ng/dL) following oral administration of testosterone esters.

### DETAILED DESCRIPTION

The following detailed description relates to embodiments according to the present invention (as defined in the claims) as well as reference embodiments disclosed herein. Any description of a method of treatment by therapy will be understood as relating to the respective product for use in the corresponding method of treatment.

It has been discovered that neither medium chain (C7-C12) low lipophilicity ester (clog P <10) nor high lipophilicity (clog P > 11.5) of the ester such as testosterone palmitate, are suitable to provide sustained, safe and effective T levels from a single administration through effective and pragmatic dosing regimens (dose, dosing frequency, dosage units) wherein most of the patients remain eugonadal for most of the time. Accordingly, it has been found for the first time that a unique dose of testosterone esters of carboxylic acids having 13 and 14 carbon-atoms can offer upon single administration of compositions and dosage forms of these T esters, adequate androgenic bioactivity, and bioavailability as compared to testosterone palmitate (C16), and maintain sustained T levels in a patient as compared to testosterone undecanoate (C11), and upon multiple dosing in a patient, result in T levels within eugonadal range for most of the time with no to very short excursions to hypogonadal levels (<300 ng/dL). Moreover, the compositions/dosage forms of these lipobalanced T13 and T14 testosterone esters at their unique daily dose when administered to a group of patients, result in steady state (after at least 7 days of dosing) T levels within eugonadal range for majority of patients with no to very short excursions to hypogonadal levels.

Unlike oral compositions of the medium chain, e.g. T11 and T12 (fast to partition out of chylomicron leading to shorter serum T level duration), and longer chain, e.g. T16 esters (too slow to partition out of chylomicron to give adequate T levels), it has been found that the oral compositions and dosages for use according to the present invention, using T13 and T14 esters provide the needed characteristics for adequate oral bioavailability of the ester, adequate rate and extent of ester partitioning in and out of the chylomicron, especially post-prandial chylomicrons in concert with chylomicrons disposition kinetics. The result is sustained clinical effectiveness observed upon a single oral administration of T esters by providing the mean serum T C_{avg t12-t24} within the desired effective eugonadal range, in most of the patients for most of the time at levels >300 ng/dL.

Furthermore, it has been discovered that T13 and T14 testosterone esters each have a unique daily dose range for which, upon daily administration to each subject in a group (of at least for example 12 hypogonadal males) for a period of at least 84 days, provides a serum testosterone C_{avg} of 300 ng/dL to 1100 ng/dL in at least 75% of the hypogonadal males in the group, and at least one of the following:
- a steady state serum T concentration of <300 ng/dL for no more than 7 hours in a 24-hour period in 50% or less of the subjects.
- a steady state serum T concentration of>300 ng/dL for at least 12-24 hours post-dosing in a 24-hour period in majority of the subjects .
- a steady state serum T concentration serum T levels of<300 ng/dL for no more than 7 hours in a 24-hour period in 50% or less subjects, 300 ng/dL for at least 12-24 hours post-dosing in a 24-hour period in majority of the subjects.
- a serum testosterone Cₘₐₓ of less than 1500 ng/dL in at least 85% of the subjects in the group;
- a serum testosterone Cₘₐₓ of about 1800 ng/dL to about 2500 ng/dL in 5% or less of the subjects in the group;
- a serum testosterone Cₘₐₓ greater than 2500 ng/dL in about 1% or less of the subjects in the group.

In addition, it has been found that the compositions of the unique T13 and T14 testosterone esters each have a distinctive daily dose range for which upon single daily dose administration, provides a steady state serum T concentration of<300 ng/dL for no more than 7 hours in a 24-hour period. The compositions of the unique T13 and T14 testosterone esters each have a unique daily dose range for which upon single daily dose administration provides longer-lasting serum T concentrations.

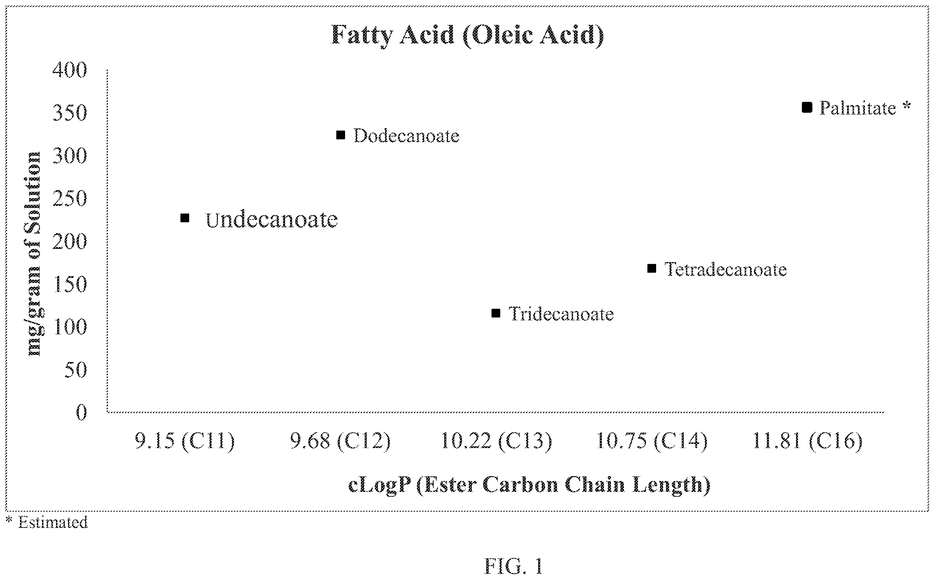

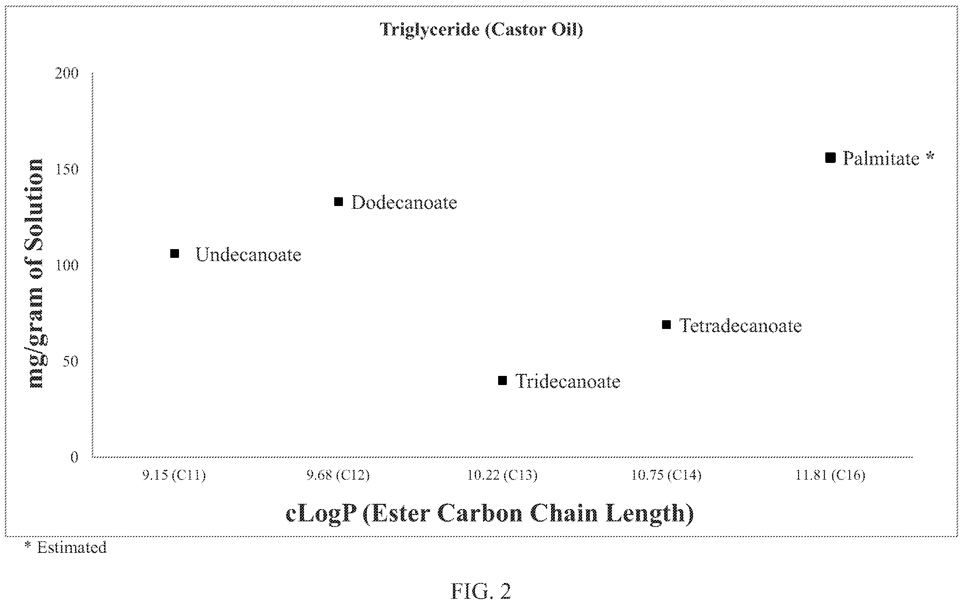

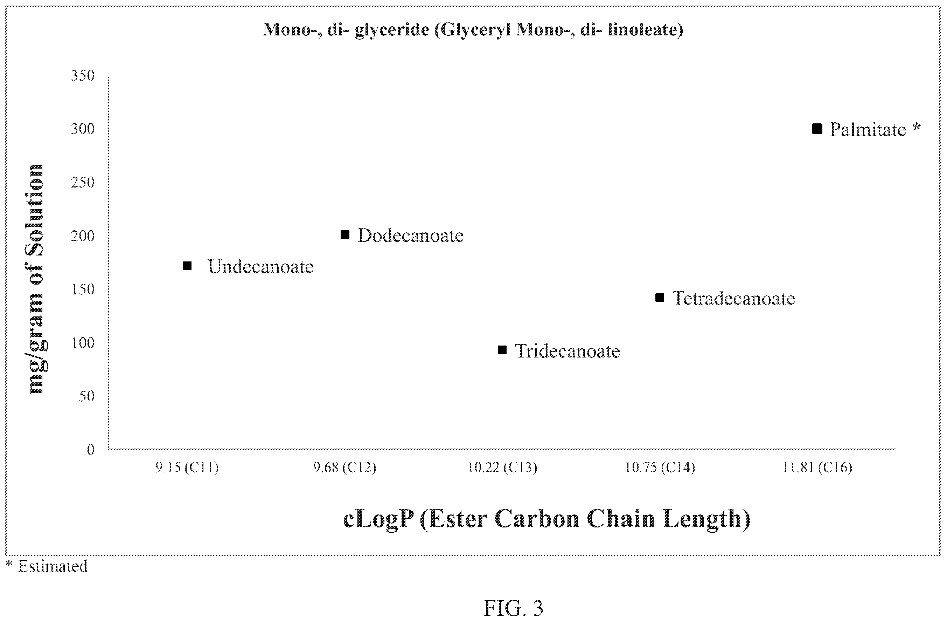

Contrary to expectations based on teachings in the art, it has been found T13 and T14 testosterone esters have unexpected lower solubility in most of the commonly desired lipid solvents (as evident from FIG.1-3) for testosterone ester oral compositions. Given its unique effective daily dose range it presents a challenge to design compositions leading to patient-friendly dosage form and dosing regimen. It has been found that oral compositions of T13 and T14 testosterone esters of this invention need not be dissolved under ambient conditions or at human body temperature, be solubilized or be in solution (e.g. at or above 30°C, or at 30° to 40°C etc.) to provide the mean serum T C_{avg t12-t24} within the desirable effective eugonadal range upon single oral administration, such that the serum T levels are sustained in most of the patients at levels >300 ng/dL for a large percentage of the dosing period with a patient-friendly regimen with lower dosing frequency administration in a day and/or with fewer number of dosage units per administration.

Accordingly, it has been discovered that by having significant not dissolved or not solubilized fraction of the T13 or T14 testosterone ester dose in the composition or dosage form for use according to the current invention, one can achieve a practical dosing regimen with adequate drug loading in the composition/dosage form that allows for adequate bioavailable testosterone levels restoration with manageable dosage units per dose and thus, an oral therapy for treatment of hypogonadism that is convenient, safe (for e.g. Cₘₐₓ no more than 1500 ng/dL), effective (for e.g. mean C_{avg t0-t24} within the eugonadal range of 300 ng/dL to 1100 ng/dL), and longer lasting (e.g. mean serum T C_{avg t12-t24} at greater than 300 ng/dL upon a single administration).

Before the present testosterone ester compositions, dosage forms and related products for use are disclosed and described, it is to be understood that this invention is not limited to the particular process steps and materials disclosed herein, but is extended to equivalents thereof, as would be recognized by those ordinarily skilled in the relevant arts. It should also be understood that terminology employed herein is used for the purpose of describing particular embodiments only and is not intended to be limiting.

It should be noted that, the singular forms "a," "an," and, "the" include plural referents unless the context clearly dictates otherwise. Thus, for example, reference to "an excipient" includes reference to one or more of such excipients, and reference to "the carrier" includes reference to one or more of such carriers.

### Definitions

As is known in the art, the term "testosterone ester" generally refers to a compound having the structure:

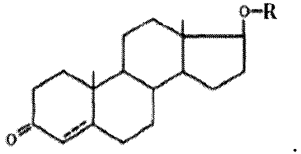

As used herein, the terms "T13 testosterone ester" or "T13 ester" or "T13" can be used interchangeably and refer to a testosterone ester, namely testosterone tridecoate, having the structure shown above, wherein -R is -C₁₃H₂₅O. Similarly, the terms "T14 testosterone ester" or "T14 ester" or "T14" can be used interchangeably and refer to a testosterone ester, namely testosterone tetradecoate, having the structure shown above, wherein R is -C₁₄H₂₇O.

As used herein, the term "treatment," when used in conjunction with the administration of pharmaceutical compositions and dosage forms containing testosterone esters (T13 ester and/or T14 ester), refers to the administration of the dosage forms (for e.g. capsule dosage form) and pharmaceutically acceptable compositions to subjects who are either asymptomatic or symptomatic. In other words, "treatment" can both be to reduce or eliminate symptoms associated with a condition present in a subject, or it can be prophylactic treatment, i.e. to prevent the occurrence of the symptoms in a subject. Such prophylactic treatment can also be referred to as prevention of the condition.

As used herein, the terms "formulation" and "composition" are used interchangeably and refer to a mixture of two or more compounds, elements, or molecules. In some aspects the terms "formulation" and "composition" may be used to refer to a mixture of one or more active agents with a carrier or other excipients. Furthermore, the term "dosage form" can include one or more formulation(s) or composition(s) provided in a format for administration to a subject. When any of the above terms is modified by the term "oral" such terms refer to compositions, formulations, or dosage forms formulated and intended for oral administration to subjects.

As used herein, the term "fatty acid" refers to unionized carboxylic acids with a long aliphatic tail (chain), either saturated or unsaturated, conjugated or non-conjugated.

Unless otherwise specified, the term Cg to C₂₂ fatty acid glycerides refers to a mixture of mono-, di-, esters of medium to long chain (C₈ to C₂₂) fatty acids.

As used herein, the term "solidifying agent" or "solidifying additive" are used interchangeably and refer to a pharmaceutically acceptable additive that is in a solid physical state at 20°C. Similarly, a "solid lipophilic additive" refers to a lipophilic compound or component that is in a solid physical state at 20°C and/or renders the composition or dosage form non-liquid, such as solid or semi-solid. As used herein, the terms "not solubilized," when used to describe the state of the T13 or T14 testosterone ester in the carrier, additive composition and/or capsule fill, dosage form, refer to the presence of some non-liquid state which is predominantly non-crystalline T13 or T14 testosterone ester.

As used herein, the terms "not fully dissolved," when used to describe the state of the T13 or T14 testosterone ester in the carrier (e.g. lipophilic additive, hydrophilic additives or combinations thereof), compositions or dosage forms for use according to the current invention, refers to the presence of non-liquid state T13 or T14 testosterone ester, predominantly as crystalline and/or non-crystalline T13 or T14 testosterone ester.

It is understood that crystalline and/or non-crystalline states can be visually assessed when observed under hot-stage microscope over a temperature of about 20°C to about 75°C; at a temperature of about 20°C; at about 25°C; at ambient room temperature; at human body temperature (e.g. about 37°C); at 30°C; above 30°C; or above 30°C, including about 30-40°C. It is also understood that crystalline states can be assessed by the presence of crystalline T13 or T14 testosterone ester melting related peak (about 60 to about75°C ) when the composition or oral dosage form is subjected to differential scanning calorimetry, or equivalent known in the art.

As used herein, the term "soluble" is as a measure or characteristic of the drug (e.g T13 to T14 testosterone ester) with regards to its ability to dissolve in a given solvent. The solubility of a T13 or T14 testosterone ester in a particular component of the composition, or in the compositions for use according to the current invention refers to the amount of the T13 or T14 testosterone ester dissolved to form a visibly clear solution at a specified temperature such as about 25°C or about 37°C.

As used herein, the term "lipophilic," refers to compounds that are not freely soluble in water; and the term "lipophilic surfactant" refers to surfactants that have HLB values of about 10 or less. Conversely, the term "hydrophilic" refers to compounds that are soluble in water; and the term "hydrophilic surfactant" refers to surfactants that have HLB values of more than about 10.

As used herein, the term "capsule fill" refers to the composition disposed in a capsule dosage form.

As used herein, "subject" refers to a mammal that may benefit from the administration of a drug composition as described herein. Examples of subjects include humans. In one aspect, the subject can be a human male. In another embodiment, the subject can be a hypogonadal male. As explained above, the human subject to be treated in accordance with the present invention is a hypogonadal male. As used herein, the testosterone deficiency or hypogonadism in a male human subject (hypogonadal male) refers to a condition wherein the average baseline plasma testosterone concentration (T-C_{avg-B}) is about 300 ng/dL or less. However in some instances, testosterone deficiency or hypogonadism in a male human subject refers to a condition wherein the average baseline plasma testosterone concentration is about 400 ng/dL or less.

A used herein, a "responder" is a subject who responds to exogenous oral T13 or T14 testosterone ester treatment or therapy. "Responder analysis" is the assessment of the effectiveness of testosterone ester (T13 and T14) therapy in a group of subjects deemed to get benefits of testosterone therapy.

As used herein, "group" or "group of subjects" refers to a collection of at least 12 human male subjects who receive and respond to exogenous oral administration of the compositions disclosed herein, namely T13 and T14 testosterone ester-containing compositions. In one aspect, the group can include at least 100 or at least 300 male subjects. In another aspect, the group can include at least 1000 male subjects. In another embodiment, the subjects can be hypogonadal subjects.

The term "oral administration" represents any method of administration in which an active agent can be administered by swallowing, chewing, or sucking of the dosage form. The composition for use according to the current inventions can be admixed with food or drink prior to being orally consumed.

As used herein, a "dosing regimen" or "regimen" such as an "initial dosing regimen" or "starting dose" or a "maintenance dosing regimen" refers to how, when, how much, and for how long a dose of the compositions for use according to the present invention can be administered to a subject. For example, an initial or starting dose regimen for a hypogonadal male subject may provide for a total daily dose of 600 mg administered in two divided doses at least 12 hours apart (e.g. once with breakfast and once with dinner) with meals having about 20-55 g of fat content repeated daily for 30 days.

As used herein, "daily dose" refers to the amount of active agent (e.g. T13 or T14 testosterone ester) administered to a subject over a 24 hour period of time. The daily dose can be administered two or more administrations during the 24 hour period. In one embodiment, the daily dose provides for two administrations in a 24 hour period. With this in mind, an "initial dose" or initial daily dose" refers to a dose administered during the initial regimen or period of a dosing regimen.

As used herein, "non-liquid" when used to refer to the state of a composition disclosed herein refers to the physical state of the composition as being a semi-solid or solid.

As used herein, "solid" and "semi-solid" refers to the physical state of a composition that supports its own weight at standard temperature and pressure, and has adequate viscosity or structure to not freely flow. Semi-solid materials may conform to the shape of a container under applied pressure.

As used herein, "titration" or "dose titration" or "dose adjustment" are used interchangeably and refer to an increase or decrease of the total daily dose of testosterone ester (T13 or T14) administered to a subject, typically based on the response of the subject to the exogenous administered testosterone undecanoate. The dose can be increased or decreased based on the measurement of serum testosterone concentration after a steady state has been achieved.

As used herein, "steady state" refers to the achievement of stable serum total testosterone levels upon a continuous dosing regimen (e.g. once daily, twice daily etc.) of the administered T13 and/or T14 testosterone ester at a given dose, after at least 7 consecutive days (typically achieved after at least 15 days), following the start of the dosing regimen. Unless otherwise stated, steady states values set forth herein refer to steady states achieved after a final dose titration (i.e., no additional titrations are required), including situations where no dose titration is required. Similarly, as used herein, the "steady state serum concentration (Cₛₛ, Css)" or "mean steady state serum concentration (mean Cₛₛ)" of testosterone refers to the achievement of a stable serum total testosterone concentration in a subject or group of subjects, respectively, in response to a continuous dosing regimen (e.g. once daily, twice daily etc.) of the administered T13 and/or T14 testosterone ester at a given dose, after at least 7 days (typically achieved after at least 15 days), following the start of the dosing regimen. It should be further noted that the when a dose adjustment (increase or decrease in total daily dose of T13 and/or T14 ester administered) is made as part of the dose-titration during the treatment, the mean Cₛₛ is achieved at least about 7 days after the initiation of the change in the dose administered.

As used herein, the terms "release" and "release rate" are used interchangeably to refer to the discharge or liberation of a substance, including without limitation a drug, from the dosage form into a surrounding environment such as an aqueous medium either *in vitro or in vivo.*

As used herein, an "effective amount" or a "therapeutically effective amount" of a drug refers to a non-toxic, but sufficient amount of the drug, to achieve therapeutic results in treating a condition for which the drug is known to be effective. It is understood that various biological factors may affect the ability of a substance to perform its intended task. Therefore, an "effective amount" or a "therapeutically effective amount" may be dependent in some instances on such biological factors. Further, while the achievement of therapeutic effects may be measured by a physician or other qualified medical personnel using evaluations known in the art, it is recognized that individual variation and response to treatments may make the achievement of therapeutic effects a somewhat subjective decision. The determination of an effective amount is well within the ordinary skill in the art of pharmaceutical sciences and medicine. See, for example, Meiner and Tonascia, "Clinical Trials: Design, Conduct, and Analysis," Monographs in Epidemiology and Biostatistics, Vol. 8 (1986).

As used herein, the term "delayed release" refers to the release into an aqueous solution of the T13 or T14 testosterone ester from the composition or oral dosage form in a time delayed manner attributed either to the inherent nature of the composition or to a coating which may surround the composition or the oral dosage form. A traditional gelatin or non-gelatin non-enteric capsule shell does not alone constitute a delayed release mechanism. In one embodiment, the delayed release is such that about 20% or less of the T13 or T14 testosterone ester is released within the first 15 minutes after the composition is contacted by the aqueous solution.

The terms "serum testosterone levels," "serum T levels," "serum testosterone concentration," "plasma testosterone concentration," "testosterone concentration in the blood," and "serum testosterone concentration," are used interchangeably and refer to the "total" testosterone concentration which is the sum of the bioavailable testosterone including free and bound testosterone concentrations. Unless otherwise specified, these values are "observed" testosterone concentrations without adjusting or correcting for the base-line serum testosterone levels in the subject(s). As with any bio-analytical measure, for increased consistency the method employed to measure initial serum testosterone levels should be consistent with the method used to monitor and re-measure serum testosterone levels during clinical testing and testosterone therapy for a subject. Unless otherwise stated, "testosterone concentration" refers to serum total testosterone concentration.

As used herein, the average serum testosterone concentration can be determined using methods and practices known in the art. For example, the average baseline plasma testosterone concentration of a human male is the arithmetic mean of the total plasma testosterone concentrations determined on at least two consecutive time points that are reasonably spaced from each other, for example from about 1 hour to about 168 hours apart. In a particular case, the plasma testosterone concentration can be determined on at least two consecutive times that are about 12 hours to about 48 hours apart. In another particular method, the plasma testosterone concentration of the human male can be determined at a time between about 5 o'clock and about 11 o'clock in the morning. Further, the plasma testosterone concentration can be the determined by standard analytical procedures and methods available in the art, such as for example, automated or manual immunoassay methods, liquid chromatography or liquid chromatography- tandem mass spectrometry (LC-MSMS) etc.

As used herein, the term AUCₜ₁₋ₜ₂ is the area under the curve of a plasma-versustime graph determined for the analyte from the time "t1 to time t2". Wherein t1 and t2 are times (in hours) post dosing. For Example, t1 could be 1 hour and t2 could be 2 hours.

As used herein, the term "C_{avg}," "Cₐᵥₑ," or "C-average" are used interchangeably, and is determined as the AUCₜ₁₋ₜ₂ mean AUC divided by the time period (|t₁-t₂|). For example, C_{avg t0-t8} is the average plasma concentration over a period of 8 hours from t₁=0 to t2=8 hours) post-dosing determined by dividing the AUC t₀₋ₜ₈ value by 8. Similarly, C_{avg t0-t12} is the average plasma concentration over a period of 12 hours post-dosing determined by dividing the AUCt₀₋ₜ₁₂ value by 12 (tl=0-t2=12). Similarly, C_{avg t12-t24} is the average plasma concentration over a period of 12 hours post-dosing determined by dividing the AUCt₁₂₋ₜ₂₄ value by 12 (t1=12-t2=24);C_{avg-t24} is the average plasma concentration over a period of 24 hours post-dosing determined by dividing the AUCt₀₋ₜ₂₄ value by 24 (t1=0-t2=24), and so on. Unless otherwise stated, all C_{avg} values are considered to be C_{avg-t24} and unless otherwise stated, all the time values are expressed in hours (h). For example, the term C_{avg t0-t24} denotes C_{avg} from time zero (0) to 24 hours post dosing.

As used herein, "Cₜ" refers to the serum concentration of testosterone at time "t" prior to or after administration of the d_{osage} for use according to the current invention. The time "t" is generally in hours, unless otherwise specified. For example, a Ct of "C_{(-2 to 0)}refers to serum testosterone concentration measured in sample collected between the time of about 2 hours before and just immediately prior to dosage administration to the subject tested. Similarly, Ct of "C₍₂ₜₒ₄₎" refers to serum testosterone concentration measured in sample collected between the time of about 2 hours and 4 hours after administration of a dosage to the subject tested.

As used herein "SIF" or "simulated intestinal fluid" refers to "intestinal fluid, simulated TS" in accordance with the USP. In one embodiment, the SIF does not contain pancreatic enzyme. In another embodiment, SIF may be a fed or fasted simulated intestinal aqueous solution comprising phosphatidyl choline and from about 2mM to 20 mM bile salts.

As used herein "SGF" or "simulated gastric fluid" refers to "Gastric fluid, Simulated TS" in accordance with the USP. In one embodiment, the SGF does not contain the enzyme pepsin. In another embodiment, the SGF may also be a simple 0.1 N HC1 solution in water.

As used herein "single unit" when used to describe dosing of a subject refers to the dosage form being a single dosage form, e.g. a single tablet, capsule, etc. In contrast, "multiple unit" when used to describe dosing of a subject refers to the dosage including two or more dosage forms, e.g. 2 tablets, 3 capsules, etc. It is noteworthy that multiple unit dosage forms generally will be the same type of dosage forms (i.e. tablet or capsule) but are not required to be the same dosage form type.

As used herein, "free of" or "substantially free of" of a particular compound or compositions refers to the absence of any separately added portion of the referenced compound or composition. Free of or substantially free of can include the presence of 1 wt% or less (based on total composition weight) of the referenced compound which may be present as a component or impurity of one or more of the ingredients.

As used herein, the term "about" is used to provide flexibility to a numerical range endpoint by providing that a given value may be "a little above" or "a little below" the endpoint. As used herein, a plurality of items, structural elements, compositional elements, and/or materials may be presented in a common list for convenience. However, these lists should be construed as though each member of the list is individually identified as a separate and unique member. Thus, no individual member of such list should be construed as a *de facto* equivalent of any other member of the same list solely based on their presentation in a common group without indications to the contrary.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a *de facto* equivalent of any other member of the same list solely based on their presentation in a common group without indications to the contrary.

Concentrations, amounts, levels and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges or decimal units encompassed within that range as if each numerical value and sub-range is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc., as well as 1, 2, 3, 4, and 5, individually. This same principle applies to ranges reciting only one numerical value as a minimum or a maximum. Furthermore, such an interpretation should apply regardless of the breadth of the range or the characteristics being described.

### Embodiments according to the Invention and Reference Embodiments

Reference will now be made in detail to preferred embodiments of the invention (as defined in the claims) as well as reference embodiments disclosed herein. While the invention will be described in conjunction with the preferred embodiments, it will be understood that it is not intended to limit the invention to those preferred embodiments. To the contrary, it is intended to cover alternatives, variants, modifications, and equivalents as may be included within the scope of the invention as defined by the appended claims.

With the above described background in mind, the inventors have identified a need for an efficient and patient-friendly oral delivery means to help restore testosterone levels in patients in need of such treatment. In particular, compositions, dosage forms and related uses have been discovered which are capable of restoring serum T levels to effective eugonadal range of>300 ng/dL in most patients for longer periods of time post dosing in large percentage patients. Further, the compositions, dosage forms and related uses disclosed herein are able to accomplish these desirable results while still providing patient-friendly regimens - such as, a practical T equivalent daily dose, less frequent administration in a day, and fewer number of dosage units per administration.

It is generally believed that in order to promote lymphatic absorption higher lipophilicity of the drug is preferred. The lipophilicity of different testosterone esters is expressed in terms of its octanol-water partition coefficient determined experimentally (LogP) or calculated using a software program (cLogP). For the straight chain esters of testosterone, the lipophilicity increases with the increasing carbon chain length of the ester moiety, as illustrated in the Table below:

| Lipophilicity of testosterone esters as a function of carbon chain length of the ester moiety | |
|---|---|
| **Testosterone Ester (abbreviation)** | **cLog P*** |
| Testosterone Propionate (T3) | 4.9 (Low) |
| Testosterone Enanthate (T7) | 7.03 (Low) |
| Testosterone Decanoate (T10) | 8.62 (Low) |
| Testosterone Undecanoate (T11) | 9.15 (Low) |
| Testosterone Dodecanoate (T12) | 9.68 (Low) |
| Testosterone Tridecoate (T13) | 10.22 (Balanced) |
| Testosterone Teradecoate (T14) | 10.75 (Balanced) |
| Testosterone Palmitate (T6) | 11.81 (High) |

| | |
|---|---|
| **Calculated using ACD*/*PhysChem Suite*™*, ACD*/*ChemSketch (FreeWare), version 12.00, Build 38526, Advanced Chemistry Development, Inc., Toronto, On, Canada, www.acdlabs.com, 2010. It is understood that absolute and differential magnitude may vary depending on methodology, but trend is expected to be same.* | |

It has been discovered that T13 and T14 testosterone esters are uniquely lipo-balanced when used alone or in combination with other testosterone esters and are uniquely able to provide higher and therapeutically safe testosterone concentrations as compared to low lipophilic medium chain fatty acid esters and high lipophilic longer chain fatty acid esters. The testosterone derived from T13 or T14 testosterone esters can enable once daily oral testosterone replacement therapy for male hypogonadism and/or sexual disorder. In contrast, "non-lipobalanced" testosterone esters (T5-T12 and T15-T16) require more than one daily administration and those administrations are at significantly higher testosterone equivalent daily doses, which may in turn cause unsafe deleterious adverse effects to the subject.

Additionally, it has been discovered that that not all prodrugs (e.g. testosterone ester) having LogP > 5 and having an oil solubility of at least 50 mg/mL are suitable for effective delivery of T ester for longer-lasting activity. It has also been found that the T13 and T14 testosterone esters need not be dissolved in a mixture comprising one or more lipophilic surfactant and one or more hydrophilic surfactant in order to provide the desired bioavailability and PK parameters. Additionally, the T13 and T14 testosterone esters can remain "not solubilized" at or above 30°C, in the components of the delivery system (for e.g. lipophilic or hydrophilic surfactants, or their mixtures) that contribute, in part to solubilizing the active ingredient.

Further, it has been found that hydrophilic surfactant (HS) can be an optional component in the compositions and dosage forms thereof as in the present invention, to achieve the serum T C_{avg t12-t24} within the desirable effective eugonadal range upon single oral administration, such that the serum T levels are sustained in most of the patients at levels >300 ng/dL for a large percentage of the dosing period with a patient-friendly regimen (i.e. practical T equivalent daily dose, less frequent administration in a day, and fewer number of dosage units per administration).

With this in mind, in one embodiment, an oral pharmaceutical composition for administration to subjects in need of testosterone is provided. The composition comprises a testosterone ester and a pharmaceutically acceptable carrier. The testosterone ester can have the structure: 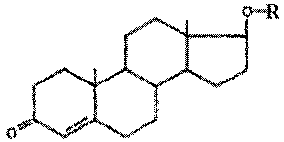 wherein R is -C₁₃H₂₅O or -C₁₄H₂₇O. One or both of the esters can be present in the pharmaceutical composition. The composition is formulated such that upon single dose administration to a group of human subjects, the composition provides a mean serum testosterone C_{avg t12-t24} that is within about 35% to about 70% of the mean serum testosterone C_{avg} t₀₋ₜ₂₄.

In another embodiment, an oral pharmaceutical composition for administration to subjects in need of testosterone therapy is provided that includes a testosterone ester and a pharmaceutically acceptable carrier. The testosterone ester can have the structure: 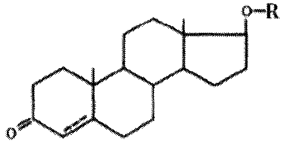 wherein, wherein R is -C₁₃H_{25O} or -C₁₄H₂₇-O. Further, the composition can include one or both of the esters. Further, the composition can be formulated such that upon administration of a daily dose of about 420 mg to about 1250 mg of the testosterone ester to each subject in a group of at least 12 hypogonadal males for a period of at least 84 days, 50% or less of the subjects in the group have a serum testosterone concentration that falls below 300 ng/dL for more than 7 hours per day at steady state. In embodiments in which the phrase "a serum testosterone concentration that falls below 300 ng/dL for more than X hours per day..." is used, it is noted that the X hours (e.g. 7 hours or 3.5 hours) can be, but need not be, consecutive.

In another embodiment, an oral pharmaceutical composition for administration to subjects in need of testosterone therapy is provided that includes a testosterone ester and a pharmaceutically acceptable carrier. The testosterone ester can have the structure: 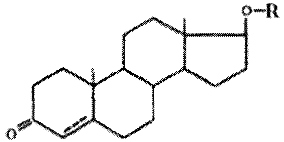 wherein, wherein R is -C₁₃H₂₅O or -C₁₄H₂₇O. Further, the composition can include one or both of the esters. Further, the composition can be formulated such upon twice-a-day administration to each subject in a group of at least 12 subjects for a period of at least 84 days, less than 20% of the subjects has a serum testosterone concentration of less than 300 ng/dL for more than 3.5 hours per day.

In another embodiment, an oral pharmaceutical composition for administration to subjects in need of testosterone therapy is provided that includes a testosterone ester and a pharmaceutically acceptable carrier. The testosterone ester can have the structure: 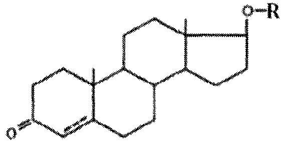 wherein, wherein R is -C₁₃H₂₅O or -C₁₄H₂₇O. Further, the composition can include one or both of the esters. Further, the composition can be formulated such upon two consecutive administrations within a 24 hour period that are administered about 12 hours apart to a human subject provides a serum testosterone concentration for the subject that falls below 300 ng/dL for no more than 7 hours within the 24 hour period.

In another embodiment, an oral pharmaceutical composition for administration to subjects in need of testosterone therapy is provided that includes a testosterone ester and a pharmaceutically acceptable carrier. The testosterone ester can have the structure: 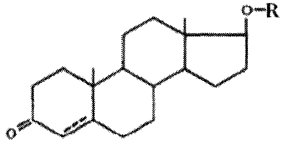 wherein, R is -C₁₃H₂₅O or -C₁₄H₂₇O. Further, the composition can include one or both of the esters. Further, the composition can be formulated such upon two consecutive administrations within a 48 hour period that are administered about 24 hours apart to a human subject provides a serum testosterone concentration for the subject that falls below 300 ng/dL for no more than 14 hours within the 48 hour period.

In an additional embodiment, a capsule dosage form for oral administration of a testosterone ester is provided. The capsule dosage form can include about 100 mg to about 400 mg of at least one testosterone ester and a lipophilic additive. The testosterone ester can have the structure: 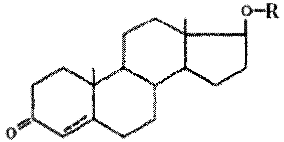 wherein R is -C₁₃H₂₅O or -C₁₄H₂₇O and one or both esters can be present in the dosage form.
The capsule dosage form can be formulated such that the testosterone ester is not fully dissolved at about 20°C in the at least one lipophilic additive.

In an additional embodiment, a capsule dosage form for oral administration of a testosterone ester is provided. The capsule dosage form can include about 100 mg to about 400 mg of at least one testosterone ester and a lipophilic additive. The testosterone ester can have the structure: 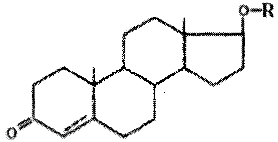 wherein R is -C₁₃H₂₅O or -C₁₄H₂₇O and one or both esters can be present in the dosage form.
The capsule dosage form can be formulated such that upon two consecutive administrations within a 24 hour period that are administered about 12 hours apart to a human subject provides a serum testosterone concentration for the subject that falls below 300 ng/dL for no more than 7 hours within the 24 hour period.

In an additional embodiment, a capsule dosage form for oral administration of a testosterone ester is provided. The capsule dosage form can include about 100 mg to about 400 mg of at least one testosterone ester and a lipophilic additive. The testosterone ester can have the structure: 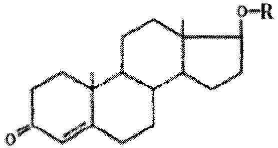 wherein R is -C₁₃H₂₅O or -C₁₄H₂₇O and one or both esters can be present in the dosage form.
The capsule dosage form can be formulated such that upon two consecutive administrations within a 48 hour period that are administered about 24 hours apart to a human subject provides a serum testosterone concentration for the subject that falls below 300 ng/dL for no more than 14 hours within the 48 hour period.

In still a further embodiment, an oral pharmaceutical composition or dosage form for use in a method of treating a human subject in need of testosterone therapy is provided. The method can include the steps of administering any of the oral pharmaceutical compositions or capsule dosage forms disclosed herein.

It is noteworthy that the discussion relating to compositional components that can be used in the oral pharmaceutical compositions is also equally applicable to the dosage form embodiments (e.g. capsule dosage form) and related methods disclosed herein unless expressly stated to the contrary. It is also noteworthy that the discussion relating to compositional components that can be used in the oral pharmaceutical compositions is also equally applicable to the tablet dosage form embodiments and related methods disclosed herein unless expressly stated to the contrary. Thus, for example, teachings regarding the use of lipophilic additives for use in the oral pharmaceutical compositions disclosed herein are also equally applicable to the capsule or tablet dosage forms and related methods described herein and vice versa.

The testosterone esters included in the compositions for use according to the present invention have the structure: 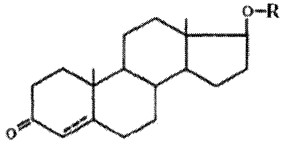 wherein R is -C₁₃H₂₅O (T13 testosterone ester) or -C₁₄H₂₇O (T14 testosterone ester). The compositions and capsule dosage forms can include one or both the esters. The exact amounts of the testosterone ester in the oral pharmaceutical composition or dosage form can vary depending on the specific testosterone ester or mixture of testosterone esters included therein. When the oral dosage compositions are formulated as oral dosage forms, such as a capsule or tablet dosage form, the dosage form can include about 100 mg to about 1250 mg of the T13 testosterone ester or the T14 testosterone ester. In a specific embodiment, when the oral dosage compositions are formulated as oral dosage forms, such as a capsule, the dosage form can include about 100 mg to about 1250 mg of the T13 testosterone ester or the T14 testosterone ester. In one embodiment, when the ester is the T13 testosterone ester and the daily dose of the ester can be about 480 mg to about 850 mg. In another embodiment, when the ester is the T14 testosterone ester and the daily dose of the ester can be from about 525 mg to about 1250 mg. In one embodiment, the testosterone ester can comprise about 0.5% to 50% by weight of the oral pharmaceutical composition or capsule dosage form. In another embodiment, the testosterone ester can comprise about 5% to 50%, or 10-50% or 15-50% or 10-35% or 10-30% or 10-25% or 15-25% or 15-30% or 20-30% or 20-25%, or more specifically, about 35%, about 30%, about 25%, about 20%, about 18%, about 16%, about 15%, about 12%, or about 10% by weight composition, of the oral pharmaceutical composition or dosage form (e.g. capsule or tablet).

The compositions a dosage forms (e.g. capsule or tablet) described herein can include a variety of pharmaceutically acceptable carriers known in the art. Non-limited examples of components that can be included as components of the pharmaceutical carrier include lipophilic surfactants, hydrophilic surfactants, triglycerides, fatty acid, or fatty acid glycerides, and combinations thereof.

In some embodiments, the pharmaceutically acceptable carrier of the composition can include a lipophilic additive. In some embodiments, the lipophilic additive can comprise at least about 50 wt% of the pharmaceutically acceptable carrier. Non-limiting examples of lipophilic additives can include lipophilic surfactants, triglycerides, tocopherol, tocopherol derivatives and combinations thereof. In one embodiment, the lipophilic additive can include a fatty acid or fatty acid glyceride. In another embodiment, lipophilic additive can include the fatty acid glyceride, and the fatty acid glyceride can be a monoglyceride, a diglyceride, or mixtures thereof. Non-limiting examples of fatty acid glycerides that can be used in the oral pharmaceutical compositions and dosage forms for use according to the present invention include monoglycerides and/or diglycerides derived from sources such as maize oil, poppy seed oil, safflower oil, sunflower oil, borage seed oil, peppermint oil, coconut oil, palm kernel oil, castor oil, and mixtures thereof. In one embodiment, the pharmaceutical composition or dosage form thereof comprises 50% by weight or less of a triglyceride. In a specific embodiment, the pharmaceutical composition or dosage form thereof, comprises less than 50 % by weight of castor oil. In another embodiment, the composition includes 10 wt% or less of triglycerides. In a further embodiment, the composition includes 5 wt% or less of triglycerides. In a still a further embodiment, the composition includes about 3 wt% or less of triglycerides. In still a further embodiment, the composition includes about 1 wt% or less of triglycerides. In another embodiment, the composition is free or substantially free of triglycerides. In another embodiment, the composition and dosage forms are free of phytosterols and phytosterol fatty acid esters.

In another embodiment, the lipophilic additive can include a lipophilic surfactant. As used herein a surfactant is considered to be a lipophilic surfactant when it has an HLB value of 10 or less. Various lipophilic surfactants can be used including, but not limited to mono-, di- glycerides of fatty acids like glyceryl monolinoleate (e.g. Maisine® 35-1), mono- and di glycerides of caprylic, capric acid (e.g. Capmul® MCM), glyceryl monooleate, reaction mixtures of alcohols or polyalcohols with a variety of natural and/or hydrogenated oils such as PEG-5 hydrogenated castor oil, PEG-7 hydrogenated castor oil, PEG-9 hydrogenated castor oil, PEG-6 corn oil (e.g. Labrafil® M 2125 CS), PEG-6 almond oil (e.g. Labrafil®M 1966 CS), PEG-6 apricot kernel oil (e.g. Labrafil®M 1944 CS), PEG-6 olive oil (e.g.Labrafil®M 1980 CS), PEG-6 peanut oil (e.g. Labrafil®M 1969 CS), PEG-6 hydrogenated palm kernel oil (e.g. Labrafil®. M 2130 BS), PEG-6 palm kernel oil (e.g. Labrafil®M 2130 CS), PEG-6 triolein (e.g. Labrafil®M 2735 CS), PEG-8 corn oil (e.g. Labrafil® WL 2609 BS), PEG-20 corn glycerides (e.g. Crovol® M40), PEG-20 almond glycerides (e.g. Crovol® A40), lipophilic polyoxyethylenepolyoxypropylene block co-polymers (e.g. Pluronic® L92, L101, L121 etc.); propylene glycol fatty acid esters, such as propylene glycol monolaurate (e.g. Lauroglycol FCC), propylene glycol ricinoleate (e.g. Propymuls), propylene glycol monooleate (e.g. Myverol P-O6), propylene glycol dicaprylate/dicaprate (e.g. Captex® 200), and propylene glycol dioctanoate (e.g. Captex® 800), propylene glycol mono- caprylate (e.g. Capryol® 90); propylene glycol oleate (e.g. Lutrol OP2000); propylene glycol myristate; propylene glycol mono stearate; propylene glycol hydroxy stearate; propylene glycol ricinoleate ; propylene glycol isostearate; propylene glycol mono-oleate; propylene glycol dicaprylate/dicaprate; propylene glycol dioctanoate; propylene glycol caprylate-caprate; propylene glycol dilaurate; propylene glycol distearate; propylene glycol dicaprylate; propylene glycol dicaprate; mixtures of propylene glycol esters and glycerol esters such as mixtures composed of the oleic acid esters of propylene glycol and glycerol (e.g. Arlacel® 186); sterol and sterol derivatives such as cholesterol, sitosterol, phytosterol, phytosterol fatty acid esters, PEG-5 soya sterol, PEG-10 soya sterol, PEG-20 soya sterol, and the like; glyceryl palmitostearate, glyceryl stearate, glyceryl distearate, glyceryl monostearate, or a combination thereof; sorbitan fatty acid esters such as sorbitan monolaurate (e.g. Arlacel 20), sorbitan monopalmitate (e.g. Span-40), sorbitan monooleate (e.g. Span-80), sorbitan monostearate, and sorbitan tristearate, sorbitan monolaurate, sorbitan monopalmitate, sorbitan monooleate, sorbitan trioleate, sorbitan sesquioleate, sorbitan tristearate, sorbitan monoisostearate, sorbitan sesquistearate, and the like; fatty acids such as capric acid, caprylic acid, oleic acid, linoleic acid, myristic acid, menthol, menthol derivatives, lecithin, phosphatidyl choline, bile salts, and the like, and mixtures thereof. It is important to note that some lipophilic surfactants may also function as the solubilizer component of the compositions and oral dosage forms.

In one embodiment, the lipophilic surfactant can be selected from the group consisting of glyceryl monolinoleate (e.g. Maisine® 35-1), mono- and di glycerides of caprylic, capric acid (e.g. Capmul® MCM), glyceryl monooleate, propylene glycol mono caprylate, propylene glycol oleate, propylene glycol monostearate, propylene glycol monolaurate, propylene glycol mono-oleate, propylene glycol dicaprylate/dicaprate, sorbitan monooleate, PEG-5 hydrogenated castor oil, PEG-7 hydrogenated castor oil, PEG-9 hydrogenated castor oil, PEG-6 corn oil, PEG-6 almond oil, PEG-6 apricot kernel oil, PEG-6 olive oil, PEG-6 peanut oil, PEG-6 hydrogenated palm kernel oil, sorbitan monolaurate (e.g. Arlacel 20), sorbitan monopalmitate, sorbitan monooleate , sorbitan monostearate, sorbitan tristearate, sorbitan monolaurate, sorbitan monopalmitate, sorbitan monooleate, sorbitan trioleate, sorbitan sesquioleate, sorbitan tristearate, sorbitan monoisostearate, and combinations thereof. In some embodiments, the lipophilic surfactants can comprise at least about 50 wt% of the total pharmaceutically acceptable carrier. It should be noted that the combinations of two or more lipophilic surfactants from the same or different classes therein are also within the scope of this invention and are together can be referred to as the lipophilic surfactant, unless otherwise stated.

In embodiments of the present invention, the oral pharmaceutical compositions or dosage forms (e.g. capsule or tablet) can include a hydrophilic additive. In one embodiment, hydrophilic additive is a selected from the group consisting of hydrophilic surfactant, celluloses - such as hydroxypropyl celluloses low molecular weight, low viscosity types (e.g. Methocel® E5, E6, E10 E15, LV100 etc. grades) and hydroxypropyl celluloses having higher molecular weight, medium to high viscosity (e.g. Methocel® K4M, K15M, K100M etc); polyvinylpyrrolidones (e.g. Kollidon k17, K30 etc); polyvinyl acetates and combinations thereof.

In one embodiment, the hydrophilic additive can be a hydrophilic surfactant. A surfactant is considered to be a hydrophilic surfactant when it has an HLB value of greater than 10. Non-limiting examples of hydrophilic surfactants include non-ionic surfactants, ionic surfactants and zwitterionic surfactants. Specifically the hydrophilic surfactants suitable for the current invention include, but not limited to alcohol-oil transesterification products; polyoxyethylene hydrogenated vegetable oils; polyoxyethylene vegetable oils; alkyl sulphate salts, dioctyl sulfosuccinate salts ; polyethylene glycol fatty acids esters; polyethylene glycol fatty acids mono- and di- ester mixtures; polysorbates, polyethylene glycol derivatives of tocopherol and the like. It should be noted that the combinations of two or more hydrophilic surfactants from the same or different classes are within the scope of this invention and are together can be referred to as the hydrophilic surfactant unless explicitly specified. In one embodiment, the hydrophilic additive can be a hydrophilic surfactant. Non-limiting examples of hydrophilic surfactants can include PEG-8 caprylic/capric glycerides, lauroyl macrogol-32 glyceride, stearoyl macrogol glyceride, PEG-40 hydrogenated castor oil, PEG-35 castor oil, sodium lauryl sulfate, sodium dioctyl sulfosuccinate, polyethylene glycol fatty acids mono- and di- ester mixtures, polysorbate 80, polysorbate 20, polyethylene glycol 1000 tocopherol succinate, phytosterols, phytosterol fatty acid esters, and mixtures thereof.

In some embodiments, surfactants utilized in the pharmaceutical compositions described herein include sterols and derivatives of sterols. In various embodiments, these surfactants are hydrophilic or lipophilic. Examples of hydrophilic sterol surfactants are lanosterol PEG-24 cholesterol ether (e.g. Solulan C-24, Amerchol), PEG-30 soya sterol (e.g. Nikkol BPS-30, from Nikko), PEG-25 phyto sterol (e.g. Nikkol BPSH-25 from Nikko), PEG-30 cholestanol (e.g. Nikkol DHC, from Nikko). Examples of Lipophilic Sterol Surfactants are Cholesterol, sitosterol, Phytosterol (e.g. GENEROL series from Henkel), PEG-5 soya sterol (e.g. Nikkol BPS-S, from Nikko), PEG-10 soya sterol (e.g. Nikkol BPS-10 from Nikko), PEG-20 soya sterol (e.g. Nikkol BPS-20 from Nikko)

In one embodiment, the oral pharmaceutical compositions or the dosage forms for use according to the current invention includes a T13 or T14 testosterone ester and a pharmaceutically acceptable carrier, wherein the T13 or T14 testosterone ester comprises about 0.5 wt% to about 50 wt% of the composition or dosage form. In another embodiment, the compositions or the dosage form of the current invention includes a T13 or T14 testosterone ester and a pharmaceutically acceptable carrier, wherein the T13 or T14 testosterone ester comprises about 5 wt% to about 50 wt% of the composition or dosage form, and wherein the carrier includes at least 50 wt% of the composition or the dosage form and wherein the testosterone ester is not solubilized at 30°C, or above 30°C, or at a temperature range above 30°C, including 30°C to about 40°C. In an additional more specific embodiment, the testosterone ester is not fully dissolved in the carrier at human body temperature.

In another embodiment, the compositions or the dosage forms for use according to the current invention includes a T13 or T14 ester and a pharmaceutically acceptable carrier, wherein the T13 or T14 testosterone ester comprises about 5 wt% to about 50 wt% of the composition or the dosage form, and wherein the carrier includes about 50 wt% to about 100 wt% of lipophilic surfactant and 0 wt% to about 50 wt% of hydrophilic surfactant. In a further embodiment, the testosterone ester is not solubilized at 30°C, or above 30°C, or at a temperature range above 30°C, including 30°C to about 40°C. In an additional more specific embodiment, the testosterone ester is not fully dissolved in the carrier at human body temperature.

In another specific embodiment, the compositions or the dosage form for use according to the current invention includes a T13 or T14 testosterone ester and a pharmaceutically acceptable carrier, wherein the T13 or T14 testosterone ester comprises about 5 wt% to about 50 wt% of the composition or the dosage form, and the carrier includes about 50 wt% to about 95 wt% a lipophilic surfactant and a hydrophilic surfactant 5 wt% to about 30 wt%. In a further more specific embodiment, the testosterone ester is not solubilized at 30°C, or above 30°C, or at a temperature range above 30°C, including 30°C to about 40°C. In an additional more specific embodiment, the testosterone ester is not fully dissolved in the carrier at human body temperature. In another more specific embodiment, the composition or the dosage form can optionally contain about 10 wt% or less of ethyl alcohol.

In one embodiment, the hydrophilic surfactant can comprise at least about 20% of the total pharmaceutical carrier. In another embodiment, the hydrophilic surfactant can comprise at least about 5 wt% of the carrier. In another embodiment, the hydrophilic surfactant can comprise less than 5 wt% of the carrier.

In another embodiment, the compositions or the dosage forms for use according to the current invention includes a T13 or T14 ester, wherein the T13 or T14 testosterone ester comprises about 5 wt% to about 50 wt% of the composition or the dosage form, and wherein the composition includes about 50 wt% to about 100 wt% of lipophilic additive and 0 wt% to about 50 wt% of hydrophilic additive. In a specific embodiment, the lipophilic additive can be lipophilic surfactant and the hydrophilic additive can be hydrophilic surfactant. In a further embodiment, the testosterone ester is not solubilized at 30°C, or above 30°C, or at a temperature range above 30°C, including 30°C to about 40°C. In an additional more specific embodiment, the testosterone ester is not fully dissolved in the lipophilic additive or the composition at human body temperature.

In one embodiment, the hydrophilic surfactant can comprise at least about 20% of the composition. In another embodiment, the hydrophilic surfactant can comprise at least about 5 wt% of the composition. In another embodiment, the hydrophilic surfactant can comprise less than 5 wt% of the composition.

In some embodiments, the oral pharmaceutical compositions or the dosage form can include both a lipophilic surfactant and hydrophilic surfactant. In one embodiment, the lipophilic surfactant and hydrophilic surfactant can be present in amounts such that the ratio of amount (wt%) of lipophilic surfactant to amount (wt%) of hydrophilic surfactant is greater than 2:1. In another embodiment, the lipophilic surfactant and hydrophilic surfactant can be present in amounts such that the ratio of amount (wt%) of lipophilic surfactant to amount (wt%) of hydrophilic surfactant is greater than 2.5:1. In another embodiment, the lipophilic surfactant and hydrophilic surfactant can be present in amounts such that the ratio of amount (wt%) of lipophilic surfactant to amount (wt%) of hydrophilic surfactant is greater than 3.5:1. In still another embodiment, the lipophilic surfactant and hydrophilic surfactant can be present in amounts such that the ratio of amount (wt%) of lipophilic surfactant to amount (wt%) of hydrophilic surfactant is at least 6.5:1.

The testosterone esters present in the oral pharmaceutical compositions and dosage forms for use according to this invention can be present in both dissolved and "not dissolved" form. For example, in one embodiment, the oral pharmaceutical composition or dosage form (e.g. capsule or tablet) can include a lipophilic additive and the testosterone ester is not fully dissolved in the lipophilic additive at 20°C. In another embodiment, the oral pharmaceutical composition or dosage form (e.g. capsule or tablet) can include a lipophilic additive and the testosterone ester is not fully dissolved in the lipophilic additive at human body temperature,. For instance, under Examples Composition Nos. 1A, 2A, and when hydrophilic surfactant is not present in the Composition Nos.14-16, 18, 20-22, all exemplify these embodiments.

In some embodiments where both the lipophilic surfactants and hydrophilic surfactants are present in the oral pharmaceutical acceptable carrier or the compositions or dosage forms (e.g. capsule or tablet) for use according to this invention, the T13 and/or T14 testosterone ester can be present such that it is not solubilized in the composition, in the pharmaceutically acceptable carrier, or in the dosage form (e.g. capsule or tablet). In one embodiment, where both the lipophilic surfactants and hydrophilic surfactants are present in the oral pharmaceutical acceptable carrier, composition, or dosage form (e.g. capsule or tablet) for use according to this invention, T13 and/or T14 testosterone ester can be present such that it is not solubilized at 30°C in the composition, in the pharmaceutically acceptable carrier, or in the dosage form. More specifically, the testosterone ester can be present such that it is not solubilized in the composition, in the pharmaceutically acceptable carrier, or in the dosage form at or above 30°C; or at a temperature above 30°C, including the 30°C to 40°C; or at human body temperature. For instance, under Examples the composition no. 1C, 2C, 5, 6, 8-13, and when hydrophilic surfactant is present in the Composition no.14-16, 18, 20-22, all exemplify these embodiments. In another embodiment, where both the lipophilic surfactants and hydrophilic surfactants are present in the oral pharmaceutical compositions or dosage form, the testosterone ester can be present such that it is not solubilized above 30°C in the composition, in the pharmaceutically acceptable carrier, or in the dosage form. In yet another embodiment, where both the lipophilic surfactants and hydrophilic surfactants are present in the oral pharmaceutical compositions or dosage form, the testosterone ester can be present such that it is not solubilized above 30°C, including 30°C to 40°C, in the composition, in the pharmaceutically acceptable carrier, or in the dosage form. In these embodiments, the dosage form can be either a capsule or a tablet.

In one aspect of the present invention, the T13 or T14 testosterone ester is not dissolved in the carrier (e.g. lipophilic additive, hydrophilic additives or combinations thereof) or compositions or dosage forms for use according to the current invention. Optionally, part of the ester is present in the liquid carrier (e.g. lipophilic additive, hydrophilic additives or combinations thereof) or compositions or dosage forms for use according to the current invention, in suspended form at normal temperature, such that the ester is not fully dissolved in the carrier (e.g. lipophilic additive, hydrophilic additives or combinations thereof) or compositions or dosage forms at body-temperature.

In another aspect of the present invention, the oral dosage forms for use according to the present invention and compositions comprising same, comprise a T13 or T14 testosterone ester not dissolved in the pharmaceutically acceptable carrier (e.g. lipophilic additive, hydrophilic additives or combinations thereof). Specifically, T13 or T14 testosterone ester is not dissolved lipophilic surfactant and a hydrophilic surfactant. In yet another aspect of the present invention, each of the components of the oral dosage form (e.g. the composition as a whole, or the pharmaceutical carrier that includes lipophilic additive or hydrophilic additive or their combinations) individually or collectively does not contribute in fully solubilizing the T13 or T14 testosterone ester.

In a further aspect of the present invention, the component of the oral dosage forms for use according to the present invention (e.g. the composition as a whole or the pharmaceutical carrier that includes lipophilic additive or hydrophilic additive or their combinations) individually or collectively does not have solubilizing characteristics to solubilize the T13 or T14 testosterone ester. It should be noted, however, that the compositions or oral dosage forms thereof for use according to the present invention comprising, for example, about 20% by weight of the T13 or T14 testosterone ester, remains "not solubilized" at or above 30°C, including in the range of 30°C to about 40°C.

In a further aspect of the present invention the term "not solubilized" herein, can be interpreted to describe state of the T13 or T14 testosterone ester within the component of the oral dosage forms and compositions for use according to the present invention, wherein the ester is not dissolved or not fully dissolved in a liquid solution. Furthermore, such a system of "not dissolved" ester can be uniformly dispersed (for e.g. by adsorption) in a solid carrier, such as silicon dioxide, calcium silicate or magnesium aluminometasilicate to obtain free-flowing powders which can be either filled into hard capsules or compressed into tablets. It should be appreciated that powders (e.g. as sachet) for reconstitution or suspension drink, and also sachet type of dosage forms can be made and are within the scope of this invention.

The oral pharmaceutical compositions and capsule dosage forms can, in some embodiments, include at least 10 wt% of an alcohol. Non-limiting examples of alcohols that can be used as solubilizers include tocopherol, ethyl alcohol, isopropanol, butanol, benzyl alcohol, ethylene glycol, glycerol, propylene glycol, butanediol, glycerol, pentaerythritol, transcutol, dimethyl isosorbide, polyethylene glycol and mixtures thereof. In one embodiment, the alcohol can be ethyl alcohol, benzyl alcohol, tocopherol, isopropyl alcohol or combinations thereof. In one embodiment, the alcohol is an alkyl alcohol, an aromatic alcohol, or a mixture thereof. In one embodiment, the alkyl alcohol is a straight chain or branched chain alcohol. In one specific embodiment, the oral pharmaceutical composition or capsule dosage form can be free of alcohol.

In addition to the T13 and T14 testosterone esters, the oral pharmaceutical compositions and dosage forms (e.g. capsule or tablet) can further include at least one additional pharmaceutically active agent or can be formulated to be co-administered with other active agents in order to treat a target condition. Non-limiting examples of additional active agents that can be included with or co-administered with the oral pharmaceutical composition or capsule oral dosage form include phosphodiesterase type 5 (PDE-5) inhibitors, such as sildenafil citrate, tadalafil, vardenafil, avanafil, lodenafil, mirodenafil, udenafil, and the like, are used to block the degradative action of phosphodiesterase type 5 enzyme on cyclic GMP in the smooth muscle cells lining the blood vessels supplying the corpus cavernosum of the penis and are frequently used to treat erectile dysfunction. Such compounds could be co-administered with the compositions and oral dosage forms for use according to the present invention in order to provide improved clinical outcomes through synergistic pharmacological action as measured by improved (sooner, better and longer lasting) erection, potency, libido, mood, body mass, etc. in males relative to administration of the testosterone or the co-administered PDE-5 alone.

Further, in addition to T13 and T14 testosterone esters, the oral pharmaceutical compositions and dosage forms (e.g. capsule or tablet) can further include borage oil, peppermint oil or mixtures thereof for increasing the metabolic stability of testosterone by decreasing the extent of DHT formation.

The testosterone ester oral pharmaceutical compositions and dosage forms (e.g. capsule or tablet) can also be co-administered with one or more other active agents such as aromatase inhibitors (for example letrozole, anastrozole, exemestane, fadrozole, vorozole, formestane etc.), dopamine agonists (for example apomorphine, bromocriptine, cabergoline, pergolide, ropinirole, rotigotine, pramipexole, fenoldopam etc.), prostaglandins (for example alprostadil), alpha blockers (for example yohimbine, phentolamine),vasodilators (for example minoxidil) and the like, for improved clinical outcomes through synergistic pharmacological action as measured by improvements in one or more of the secondary sexual characteristics in males such as sexual activity, potency, libido, erection etc., mood, body mass and the like, relative to administration of either the testosterone or the co-administered active agent alone. In one embodiment, the additional pharmaceutical agent can be another testosterone form including, but not limited to testosterone, testosterone cypionate, testosterone buciclate, testosterone propionate, testosterone phenylpropionate, testosterone isocaprate, testosterone decanoate, testosterone undecanoate, testosterone dodecanoate and combinations thereof.

In another aspect of the invention, the oral pharmaceutical compositions and/or capsule dosage forms, namely the capsule fill, can include a solidifying agent. A solidifying agent is a pharmaceutically acceptable additive that is in a solid physical state at 20°C. Typically solidifying agents facilitate the solidification of the pharmaceutical compositions for use according to the present invention at temperatures around room temperature. The compositions and capsule fill of the present invention, including those with solidifying agents, can be non-liquid at standard temperature and pressure. In one embodiment, the composition and capsule fill can be semi-solid at standard temperature and pressure. In yet another embodiment, the composition and capsule fill can be solid at standard temperature and pressure. When present, the solidifying agent can comprise from about 0.1 wt% to about 25 wt% of the pharmaceutical composition or capsule dosage form. In another embodiment, the solidifying agent can comprise about 2 wt% to about 20 wt% of the composition or capsule dosage form. In yet a further embodiment, the solidifying agent can comprise about 3 wt% to about 15 wt% of the composition or capsule dosage form. In still a further embodiment, the solidifying agent can comprise about 3 wt% to about 9 wt% of the capsule fill. In yet a further embodiment, the solidifying agent can comprise 6 wt% to 9 wt% of the capsule fill. In one embodiment, the solidifying agent can melt at a temperature of about 45°C to about 75°C. Non-limiting examples of solidifying agents that can be used include polyethylene glycols; sorbitol; gelatin; stearic acid; cetyl alcohol; cetosterayl alcohol; paraffin wax; polyvinyl alcohol; glyceryl stearates; glyceryl distearate; glyceryl monostearate; glyceryl palmitostearate; glyceryl behenate; waxes; hydrogenated castor oil; hydrogenated vegetable oil; bees wax, microcrystalline wax; sterols; phytosterols; phytosterols fatty acid esters, cholesterol and mixtures thereof. In one embodiment, the solidifying agent includes a polyethylene glycol (PEG) having molecular weight from about 1000 to about 20,000 and their mixtures. In another embodiment the solidifying agent includes one or more selected from the group consisting of polyethylene glycol; gelatin; stearic acid; polyvinyl alcohol; glyceryl stearates; glyceryl distearate; glyceryl monostearate; glyceryl palmitostearate; hydrogenated castor oil; hydrogenated vegetable oil and cholesterol. In one embodiment, the pharmaceutical composition can be a solid at about 20°C. In yet a further embodiment, the "not dissolved" crystalline T13 and/or T14 testosterone ester can act as a solidifying agent.

The compositions and the dosage forms (e.g. capsule or tablet) for use according to the current invention can also include one or more of other additives selected from binders, bufferants, diluents, disintegrants, flavors, colorants, taste-masking agents, resins, pH modifiers, lubricants, glidants, thickening agent, opacifying agent, humectants, desiccants, effervescing agents, plasticizing agents and the like.

The oral compositions for use according to the present invention can be formulated to take any dosage form commonly known in the pharmaceutical arts such as granules, tablet or capsule. In one embodiment the oral pharmaceutical compositions for use according to the present invention can be formulated as oral dosage forms such as capsules or tablets. In one embodiment, the oral dosage form can be a capsule having a pharmaceutical composition for use according to the present invention disposed therein. Both soft and hard gelatin and non-gelatin capsules can be used. The capsule size can be any size known in the art and can vary depending on the desired dosage amount. For instance, in one embodiment, the capsule can be a hard gelatin capsule having a fill volume of about 0.25 mL to about 1.1 mL. Similarly, in another embodiment, the capsule can be a soft gelatin capsule having a fill volume of about 0.25 mL to about 1.5 mL.

In a specific embodiment, the compositions for use according to the current invention can be formulated in the form of granules, powder mixtures or tablets. In a specific embodiment, the T13 and/or T14 present in the dosage form can be present in the form of nanoparticles or amorphous particles, or a mixture of both. In another specific embodiment, the T13 and/or T14 present in these dosage form can be present in the form of crystalline, non-crystalline or amorphous particles or a mixtures thereof having an average particle size of about 2000 nm or less, 1500 nm or less, 1000 nm, 800 nm or less, 600 nm or less, 500 nm or less, 400 nm or less, 300 nm or less, 250 nm or less, 200 nm or less, 100 nm or less, 50 nm or less, or 25 nm or less; or the average particle size of said crystalline, non-crystalline or amorphous particles or a mixtures thereof is in the range 10 nm to 2000 nm, 10 nm to 1500 nm, 10 nm to 1000 nm, 10nm to 800 nm, 10 nm to 750 nm; 10 nm to 600 nm, 10 nm to 500 nm, 10 nm to 400 nm, 10 nm to 300 nm, 10 nm to 250 nm, 10 nm to 200 nm, or 10 nm to 100 nm.

In another specific embodiment, a solution of the T13 and/or T14 testosterone esters in a carrier (e.g. lipophilic additive or hydrophilic additive or combinations thereof). Such solutions can be dispersed (e.g. by adsorption) in a solid carrier such colloidal silicon dioxide, lactose, calcium silicate, magnesium aluminum silicates, microcrystalline cellulose or combinations thereof, etc., and prepared as powder mixtures or granules or pellets to be disposed/filled into capsules or sachets, or admixed with tableting aids and compressed as tablets. Such sachets, capsules or tablets can also be formulated to contain an additional amount of the respective testosterone ester in crystalline and/or non-crystalline form, such that in the final composition or dosage form the total ester amount exists as a combination of at least two of the forms including solution, crystalline and non-crystalline forms, at about 20°C or at about human body temperature or at 30°C or above 30°C including the range 30°C to 40°C. In a further embodiment, these dosage forms provide serum testosterone levels and the pharmacokinetic parameters disclosed herein for the T13 and T14 testosterone esters upon single administration or two consecutive administrations or upon steady state.

In a further embodiment, the oral pharmaceutical composition can be formulated as dosage (e.g. capsule or tablet) form to be administered to provide a daily T13 or T14 testosterone ester dose of about 420 mg to about 1250 mg based on single unit or multiple unit dosing. In a specific embodiment, a single unit dosing comprises administering the entire required dose of the ester per administration time in the form of one unit dosage form; whereby the subject has to consume one unit dosage from per administration. In another specific embodiment, a multiple unit dosing comprises administering the entire required dose of the ester per administration time in the form of two or more unit dosage form; whereby the subject has to consume two, three, four or more unit dosages, per administration.

The dosage forms (e.g. capsule or tablet) can be immediate release, extended release, targeted release, enteric release, delayed release dosage form or combinations thereof. When formulated as oral dosage forms, including the disclosed capsule or tablet dosage forms, the dosage forms can be formulated for once-a-day administration or for twice-a-day administration. The compositions and oral dosage forms can also be formulated for administration with a meal, including once-a-day administration with a meal. While the compositions dosage forms disclosed herein can be administered with a meal, a meal is not necessarily required.

In another embodiment, the compositions and dosage forms for use according to this invention containing the T13 testosterone ester when subjected to in vitro dissolution testing using USP type 2 apparatus in about 1000 mL aqueous medium, the T13 testosterone ester releases substantially all (>90%) of the T13 testosterone ester amount comprised therein, in about 4 hours. In one embodiment, about 15% or less of the T13 testosterone ester amount present in the composition is released in the first 15 minutes. In another embodiment, about 25% or less of the T13 testosterone ester amount present in the composition is released in the first 30 minutes. In another specific embodiment, about 60% or less of the T13 testosterone ester amount present in the composition is released in the first 60 minutes. In another embodiment, about 90% or less of the T13 testosterone ester amount present in the composition is released in the first 120 minutes. In another embodiment, substantially all (>90%) of the T13 testosterone ester amount present in the composition is released in about 2 to about 4 hours.

In another embodiment, the compositions and dosage forms for use according to this invention containing the T13 testosterone ester when subjected to in vitro dissolution testing using USP type 2 apparatus in about 1000 mL aqueous medium, shows the T13 testosterone ester release profile such that about 15% or less is released in 30 minutes; about 60% or less is released in 60 minutes; about 90% or less is released in 120 minutes. In another embodiment, the compositions and dosage forms for use according to this invention containing the T13 testosterone ester when subjected to in vitro dissolution testing using USP type 2 apparatus in about 1000 mL 8% Triton X100 solution in water, the testosterone tridecanoate release profile is as follows: at least 25% lower at about 30 minutes and 60 minutes, and at least 10% lower at about 120 minutes, compared to that observed at the corresponding release time points from an identical dosage form comprising an equivalent amount of testosterone as testosterone undecanoate when treated in vitro in the same way.

Similarly, in another embodiment, the compositions and dosage forms disclosed herein containing the T14 testosterone ester, when subjected to in vitro dissolution testing using USP type 2 apparatus in about 1000 mL aqueous medium, the composition and dosage forms release substantially all (>90 wt%) of the T14 testosterone ester amount in the composition or dosage form in about 2 hours. In one embodiment, about 90 wt% or less of the T14 testosterone ester amount comprised therein is released from the composition or dosage form in the first 15 minutes. In another embodiment, substantially all (>90%) of the T13 ester in the composition or dosage form is released in about 1-2 hours.

It should be noted that the aqueous medium for the above mentioned in vitro release testing medium can be any one of the following media including about 4% to 8% (w/v). of Triton X100 solution in water, or 0.5% (w/v) to 2.5% (w/v) sodium lauryl sulphate solution in water, simulated gastric fluid, or simulated intestinal fluid.

In one embodiment, the composition or dosage form (e.g. capsule or tablet) can be administered with a meal, such as a meal that provides about 200 to about 1000 calories of energy of which 20-35% come from fats in the meal. In another embodiment, the composition or the dosage form can be administered with a standard meal. In another embodiment, the composition or capsule dosage form can be administered with a meal that provides about 50% of the calories derived from the fat. In another embodiment, the composition or the dosage form can be administered with a high-fat, high calorie meal. In another embodiment, the composition or the dosage form can be administered with a meal that provides about 500 to about 1000 calories of energy. In another embodiment, the composition or the dosage form can be administered with a meal that provides about 400 to about 700 calories derived from the fat therein. The compositional make-up of the meals that are administered can vary depending on the tastes and dietary needs of a subject. However, in some situations it may be beneficial to administer the compositions and oral dosage forms with meals that provide no fat or up to about 50 g of fat. In one embodiment, the meal can provide about 10 g to about 50 g of fat. In yet a further embodiment, the meal can provide about 20-35g of fat.

In another embodiment, the composition or the dosage form can be administered orallyto a subject, along with a meal such as breakfast, snack, food, lunch, dinner etc. In a specific embodiment, the meal can comprise about 15-55% fat. In another specific embodiment, the meal can comprise about 20-35% fat. In another specific embodiment, the meal can comprise about 20-55% fat. In another specific embodiment, the meal can comprise about 15-55% fat. In a specific embodiment, the compositions and the dosage forms containing T13 or T14 testosterone esters for use according to the current invention can enable to provide the said pharmacokinetic benefits to a subject when administered orally along with meal containing about 35g ± 20g fat content. In another embodiment, the serum T pharmacokinetic benefit provided by the T13 and T14 testosterone ester compositions and dosage forms for use according to this invention when administered with a meal containing about 30% to 35% fat is not statistically significantly different compared that when administered with a meal containing as low as 15% to 20% fat or a meal containing as high as 50% to 55% fat.

The oral pharmaceutical composition or the oral dosage forms (e.g. capsule or tablet) can be formulated to provide specific desirable pharmacokinetic outcomes. In one embodiment, upon single dose administration to a group of hypogonadal males, the composition or the dosage form provides a mean serum testosterone C_{avg t0-t24}per mg testosterone equivalent administered of at least 1.2 ng/dL/mg. In another embodiment, upon single dose administration to a group of hypogonadal males, the composition or the dosage form provides a mean serum testosterone C_{avg t0-t24} per mg testosterone equivalent administered of about 2.2 ng/dL/mg or less. In another embodiment, upon single dose administration to a group of hypogonadal males, the composition or the dosage form provides a mean serum testosterone C_{avg t0-t24} per mg testosterone equivalent administered of at about 1.2 ng/dL/mg to about 2.2 ng/dL/mg. In still a further embodiment, upon single dose administration to a group of hypogonadal males, the composition or the dosage form provides a mean serum testosterone Cₘₐₓ per mg testosterone equivalent administered of no greater than about 5.5 ng/dL/mg. In another embodiment, upon single dose administration to a group of hypogonadal males, the composition or the dosage form provides a mean serum testosterone Cₘₐₓ per mg testosterone equivalent administered of no less than about 1.4 ng/dL/mg. In an additional embodiment, upon single dose administration to a group of hypogonadal males, the composition or the dosage form provides a ratio of mean serum testosterone C_{avg 10-112} to the mean serum testosterone C_{avg t12-t24} of about 1:0.7 to about 1:1.5.

In another embodiment, oral pharmaceutical composition or dosage form (e.g. capsule or tablet) can be formulated such that when the testosterone ester is the T13 testosterone ester, upon single dose administration to a group of hypogonadal males, the composition or the dosage form can provide a mean serum testosterone C_{avg t0-t24} per mg testosterone equivalent administered of at least 1.5 ng/dL/mg and less than about 2.2 ng/dL/mg. In another embodiment, the oral pharmaceutical composition or the dosage form can be formulated such that, wherein the testosterone ester is the T13 testosterone ester, upon single dose administration to a group of hypogonadal males, the composition or the dosage form provides a mean serum testosterone Cₘₐₓ to per mg testosterone equivalent administered of about 2.9 ng/dL/mg to about 4.5 ng/dL/mg. In another embodiment, the oral pharmaceutical composition or the dosage form can be formulated such that, wherein the testosterone ester is T14 testosterone ester, upon single dose administration to a group of hypogonadal males, the composition or the dosage form provides a mean serum testosterone C_{avg t0-t24} per mg testosterone equivalent administered of at least 1.4 ng/dL/mg and less than about 1.8 ng/dL/mg. In another embodiment, the oral pharmaceutical composition or the dosage form can be formulated such that, wherein the testosterone ester is T14 testosterone ester, upon single dose administration to a group of hypogonadal males, the composition or the dosage form provides a mean serum testosterone Cₘₐₓ per mg testosterone equivalent administered of about 1.4 ng/dL/mg to about 2.8 ng/dL/mg.

In a further embodiment, the oral pharmaceutical composition or dosage form (e.g. capsule or tablet) can be formulated such that wherein upon a single dose administration to a group of human subjects it provides a mean serum testosterone C_{avg t12-t24} that is within 35% to 70% of the mean serum testosterone C_{avg 10-124}.

In another embodiment, the oral pharmaceutical composition or the dosage form can be formulated such that upon two consecutive administrations within a 24 hour period that are administered about 12 hours apart to a human subject provides a serum testosterone concentration for the subject that falls below 300 ng/dL for no more than 7 hours within the 24 hour period. It is noted that when discussing embodiments herein similar to the one set forth above, the term "the 24 hour period" refers to the total of the 12 hours post-administration time following the first dose and the 12 hours post-administration time following the second dose. In other words, the 24 hour period begins upon the administration of the first of the two consecutive doses (the second dose being administered about 12 hours following the first dose).

In another embodiment, the oral pharmaceutical composition or dosage form can be formulated such that upon two consecutive administrations within a 48 hour period that are administered 24 hours apart to a human subject provides a serum testosterone concentration for the subject that falls below 300 ng/dL for no more than 14 hours within the 48 hour period. It is noted that when discussing embodiments herein similar to the one set forth above, the term "the 48 hour period" refers to the total of the 24 hours post-administration time following the first dose and the 24 hours post-administration time following the second dose. In other words, the 48 hour period begins upon the administration of the first of the two consecutive doses (the second dose being administered about 24 hours following the first dose).

In another embodiment, the oral pharmaceutical composition or dosage form can be formulated such that upon continuous once-a-day administration to each subject in a group of at least 12 subjects for a period of at least 84 days, 50% or less of the subjects in the group have a steady state serum testosterone concentration that falls below 300 ng/dL for more than 7 hours per day.

In another embodiment, the oral pharmaceutical composition or dosage form can be formulated such that upon continuous once-a-day administration to each subject in a group of at least 12 subjects for a period of at least 84 days, 25% or less of the subjects in the group have a steady state serum testosterone concentration that falls below 300 ng/dL for more than 7 hours per day.

In another embodiment, the oral pharmaceutical composition or dosage form can be formulated such that upon continuous twice daily administration to each subject in a group of at least 12 subjects for a period of at least 84 days, less than 50% of subjects in the group have a steady state serum testosterone concentration that falls below 300 ng/dL for more than 3.5 hours per day.

In another embodiment, the oral pharmaceutical composition or dosage form can be formulated such that upon continuous twice daily administration to each subject in a group of at least 12 subjects for a period of at least 84 days, less than 20% of subjects in the group have a steady state serum testosterone concentration that falls below 300 ng/dL for more than 3.5 hours per day.

In another embodiment, the oral pharmaceutical composition or dosage form can be formulated such that upon continuous twice daily administration to each subject in a group of at least 12 subjects for a period of at least 84 days, less than 10% of subjects in the group have a steady state serum testosterone concentration that falls below 300 ng/dL for more than 3.5 hours per day.

In yet another embodiment, the oral pharmaceutical composition or dosage form can be formulated such that upon continuous twice daily administration to each subject in a group of at least 12 subjects for a period of at least 84 days, no subject in the group has a steady state serum testosterone concentration that falls below 300 ng/dL for more than 3.5 hours per day.

In another embodiment, the oral pharmaceutical composition or dosage form (e.g. capsule or tablet) can be formulated such that upon continuous once or twice daily administration to each subject in a group of at least 12 hypogonadal males for a period of at least 84 days, the dosage form provides a steady state serum testosterone C_{avg} of 300 ng/dL to 1100 ng/dL in at least 75% of the subjects in the group, and at least one of the following: 1) a serum testosterone Cₘₐₓ of less than 1500 ng/dL in at least 85% of the subjects in the group; 2) a serum testosterone Cₘₐₓ of about 1800 ng/dL to about 2500 ng/dL in 20% or less of the subjects in the group; and 3) a serum testosterone Cₘₐₓ greater than 2500 ng/dL in about 1% or less of the subjects in the group.

As discussed above, an oral pharmaceutical composition or dosage form for use in a method of treating a human subject in need of testosterone therapy is also provided. The method can include the steps of administering any of the oral pharmaceutical compositions or dosage forms (e.g. capsule or tablet) disclosed herein. The oral pharmaceutical compositions and the dosage forms can be used to treat any condition associated with testosterone deficiency, including complete absence, of endogenous testosterone in male or female subjects. Examples of conditions associated with testosterone deficiency that can be treated using the dosage forms (e.g. capsule or tablet) and/or compositions include, but are not limited to congenital or acquired primary hypogonadism, hypogonadotropic hypogonadism, cryptorchidism, bilateral torsion, orchitis, vanishing testis syndrome, orchidectomy, Klinefelter's syndrome, post castration, eunuchoidism, hypopituitarism, endocrine impotence, infertility due to spermatogenic disorders, impotence, male sexual dysfunction (MSD) including conditions such as premature ejaculation, erectile dysfunction, decreased libido, and the like, micropenis and constitutional delay, penile enlargement, appetite stimulation, testosterone deficiency associated with chemotherapy, testosterone deficiency associated with toxic damage from alcohol, testosterone deficiency associated with toxic damage from heavy metal, osteoporosis associated with androgen deficiency, and combinations thereof.

Other conditions that can be treated by the compositions and oral dosage forms disclosed herein include idiopathic gonadotropin, LHRH deficiency, or pituitary hypothalamic injury from tumors, trauma, or radiation. Typically, these subjects have low serum testosterone levels but have gonadotropins in the normal or low range. In one embodiment, the compositions or oral dosage forms may be used to stimulate puberty in carefully selected males with clearly delayed puberty not secondary to pathological disorder. In another embodiment, the compositions and oral dosage forms may be used in female-to-male transsexuals in order to maintain or restore male physical and sexual characteristics including body muscle mass, muscle tone, bone density, body mass index (BMI), enhanced energy, motivation and endurance, restoring psychosexual activity etc. In some embodiments, the T13 and/or T14 testosterone ester-containing compositions and the dosage forms thereof can be useful in providing hormonal male contraception. In some embodiments, the T13 and/or T14 testosterone ester-containing compositions and the dosage forms thereof can be used to provide treatment of one or more symptoms associated with female sexual dysfunction, anorgasmia, osteoarthritis, hormonal male contraception.

Additionally, the T13 or T14 testosterone ester-containing compositions and the dosage forms thereof can be used to treat and/or improve the patient-related outcomes including the quality of life and wellbeing of the subjects suffering from deficiency of endogenous testosterone. In some embodiments, the T13 or T14 testosterone ester-containing compositions and the dosage forms thereof can be used to treat or improve the symptoms of subjects suffering from conditions such as decreased libido, diminishing memory, anemia due to marrow failure, renal failure, chronic respiratory or cardiac failure, steroid-dependent autoimmune disease, muscle wasting associated with various diseases such as AIDS, preventing attacks of hereditary angioedema or urticaria; andropause, and palliating terminal breast cancer. In some situations, certain biomarkers such as for example, increased SHBG levels, can be used to diagnose a subject who may be in need of testosterone therapy. These biomarkers can be associated with conditions/disease states such as anorexia nervosa, hyperthyroidism, hypogonadism, androgen insensitivity/ deficiency, alcoholic hepatic cirrhosis, primary biliary cirrhosis, and the like.

In some embodiments, the compositions and dosage forms of the T13 or T14 testosterone esters improve at least one of biological absorption and metabolic stability of the testosterone ester. In one embodiment, the biological absorption of the T13 or T14 testosterone ester is intestinal lymphatic absorption.

In some embodiments, the oral compositions and dosage forms (e.g. capsule or tablets) provide for administering T13 or T14 testosterone ester by finely adjusting the total testosterone equivalent dose administered such that various desired serum testosterone levels can be provided in individual subjects along with maintaining or controlling normal physiological levels of dihydrotestosterone (DHT). In one embodiment, the oral compositions and dosage forms provide for maintaining or controlling physiological levels of DHT in a subject in need of testosterone therapy such that the physiological levels of DHT are normal or near normal and supra-physiological levels of DHT are avoided by such control or maintenance.

In another embodiment, a the compositions or dosage having a combination of T13 and T14 testosterone esters can be administered for maintaining or controlling physiological levels of DHT in a subject in need of testosterone therapy. In a further embodiment, the compositions or dosage having T13 or T14 testosterone ester can be administered in combination with testosterone and/or other testosterone ester (for e.g. testosterone undecanoate) for maintaining or controlling physiological levels of DHT in a subject in need of testosterone therapy. In a further embodiment, the compositions or dosage can have at least one of the immediate release, modified release and targeted delivery properties in various regions of the GI tract and can be administered for maintaining or controlling physiological levels of DHT in a subject in need of testosterone therapy.

In some embodiments, the oral compositions and dosage forms (e.g. capsule or tablets) containing the T13 or T14 testosterone ester for use according to the current invention upon administration for at least 7 days to subjects in need of testosterone therapy, does not result in statistically significant change from the baseline in the levels of the liver enzymes such as fractionated alkaline phosphatase, SGOT,/AST, SGPT/ALT, GGT compared to administration of placebo compositions (without T13 or T14 testosterone ester) administered for identical duration and under identical conditions. It is noteworthy that the baseline levels are based on at least two consecutive determinations prior to the start of the administration (or treatment) of the T13 and/or T14testosterone -containing compositions/dosage forms for use according to the current invention or the corresponding placebo compositions/dosage form.

Similarly, in another embodiment, the oral compositions and dosage forms (e.g. capsule or tablets) containing the T13 and/or T14 testosterone ester for use according to the current invention upon administration for at least 7 days to subjects in need of testosterone therapy, does not result in statistically significant change from the baseline in the levels of the serum LDL (low density lipoprotein) compared to administration of placebo compositions (without T13 and T14 testosterone ester) administered for identical duration and under identical conditions. It is noteworthy that the baseline levels are based on at least two consecutive determinations prior to the start of the administration (or treatment) of the T13 and/or T14testosterone -containing compositions/ dosage forms for use according to the current invention or the corresponding placebo compositions.

Subjects that can be treated by the T13 and/or T14 testosterone ester-containing compositions and dosage form of the present disclosure can be any human male in need thereof. In particular, in one embodiment, the human male may be at least 14 years of age. In another embodiment, the human male is an adult of at least age 30. In a further embodiment, the subject can be an adult male of at least age 50. In yet a further embodiment, the subject can be an adult male of at least age 60. Subjects that can be treated by the T13 and/or T14 testosterone ester-containing compositions and dosage form of the present disclosure can be any human female in need thereof. In particular, in one embodiment, the human female may be at least 14 years of age. In another embodiment, the human female is an adult of at least age 30. In a further embodiment, the subject can be an adult female of at least age 50. In a further embodiment, the subject can be an adult female who has deficient in the endogenous serum testosterone levels. In a further embodiment, the subject can be an adult female who has undergone unilateral or bilateral oophorectomy. In yet a further embodiment, the subject can be an adult female who has undergone unilateral or bilateral oophorectomy. In yet another embodiment, the subject can be a post-menopausal woman.

As discussed before, the compositions and the dosage forms for use according to the current invention comprise a testosterone ester having the chemical structure as shown below: 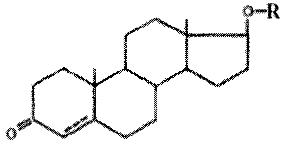 In one embodiment, the composition for use can be such that wherein the R of the testosterone ester is -C₁₃H₂₅O and when the administration to each subject in a group of hypogonadal males is continuous once-a-day for a period of at least 84 days, the administration is such that less than 50% of the hypogonadal males have a steady state serum testosterone <300 ng/dL for more than 7 hours per day when the total daily testosterone ester dose administered is about 420 mg to about 850 mg. In one embodiment, the composition for use can be such that wherein the R is -C₁₃H₂₅O and when the administration to each subject in a group of hypogonadal males is continuous once-a-day for a period of at least 84 days, the administration is such that less than 50% of the hypogonadal males have a steady state serum testosterone <300 ng/dL for more than 7 hours per day when the total daily testosterone ester dose administered is about 420 mg to 850 mg. In one embodiment, the composition for use can be such that wherein the R is -C₁₄H₂₇O and when the administration to each subject in a group of hypogonadal males is continuous once-a-day for a period of at least 84 days, the administration is such that less than 50% of the hypogonadal males have a steady state serum testosterone <300 ng/dL for more than 7 hours per day when the total daily testosterone ester dose administered is about 525 mg to 1250 mg.

In one embodiment, the composition for use can be such that wherein the R is - C₁₃H₂₅O and when the daily dose of 420 mg to 850 mg testosterone ester is administered continuous once-a-day to each subject in a group of hypogonadal males for a period of at least 84 days, the administration is such that at least 75% of the hypogonadal males in the group have a serum testosterone C_{avg} of about 300ng/dL to about 1100 ng/dL, and at least one of the following: 1) a serum testosterone Cₘₐₓ of less than 1500 ng/dL in at least 85% of the subjects in the group; 2) a serum testosterone Cₘₐₓ of about 1800 ng/dL to about 2500 ng/dL in 10% or less of the subjects in the group; and 3) a serum testosterone Cₘₐₓ greater than 2500 ng/dL in about 5% or less of the subjects in the group. In one embodiment, the composition for use can be such that wherein the R is -C₁₄H₂₇O and when the daily dose of 525 mg to 1250 mg testosterone ester is administered continuous once-a-day to each subject in a group of hypogonadal males for a period of at least 84 days, the administration is such that at least 75% of the hypogonadal males in the group have a serum testosterone C_{avg} of about 300ng/dL to about 1100 ng/dL, and at least one of the following: a serum testosterone Cₘₐₓ of less than 1500 ng/dL in at least 85% of the subjects in the group; a serum testosterone Cₘₐₓ of about 1800 ng/dL to about 2500 ng/dL in 10% or less of the subjects in the group; and a serum testosterone Cₘₐₓ greater than 2500 ng/dL in about 5% or less of the subjects in the group.

### EXAMPLES

The following examples are provided to promote a more clear understanding of certain embodiments of the present invention, and are in no way meant as a limitation thereon.

### EXAMPLE 1 - Testosterone ester compositions

Testosterone ester-containing compositions were prepared including the testosterone ester having the structure: 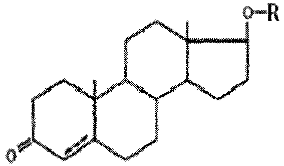 wherein, R is at least one selected from the groups -C₁₃H₂₅O (Testosterone tridecoate, T13 testosterone ester) and -C₁₄H₂₇O (Testosterone tetradecoate, T14, T14 testosterone ester). It is to be noted that 1.68 milligram (mg) of the T13 ester or 1.73 mg of the T14 testosterone ester is equivalent to 1 mg of testosterone.

Tables 1 and 1A show the typical components and their relative proportions that can be utilized in the compositions of the present inventions having the testosterone esters set forth above.

**Table 1:**

| | **Composition (weight %)** | |
|---|---|---|
| **Composition No.** | **1** | **2** |
| Component | | |
| Testosterone tridecoate, (T13) | 10-30 | - |
| Testosterone tetradecoate, (T14) | - | 10-30 |
| Carrier | 50-90 | 50-90 |
| Adjuvant* | q.s. 100 | q.s. 100 |

| | | |
|---|---|---|
| * Optional | | |

**Table 1A: Carrier components for compositions 1 and 2 of Table 1**

| | **Carrier component (weight %)** | | | | | |
|---|---|---|---|---|---|---|
| **Composition No.** | **1A** | **1B** | **1C** | **2A** | **2B** | **2C** |
| Carrier component | | | | | | |
| Lipophilic additive [e.g. Triglyceride, lipophilic surfactant, tocopherol derivative, etc.] | 100 | - | 5-95 | 100 | - | 5-95 |
| Hydrophilic additive [e.g. Hydrophilic surfactant,] | - | 100 | 5-95 | - | 100 | 5-95 |

### EXAMPLE 2 - Comparative pharmacokinetic study oftestosterone esters

Some of the compositions of the current invention having T13 and T14 testosterone ester, and compositions containing other testosterone esters, are administered to human subjects as a single dose of the esters to subjects. Serial blood samples were drawn at predetermined time (e.g. t=0, 12, 24, etc.) and analyzed for testosterone concentration using a validated HPLC-MS/MS analytical method. The Cₘₐₓ, C_{avg t1-t2}, Tₘₐₓ and AUCₜ₁₋ₜ₂ are calculated for testosterone in the serum of the subjects. Pharmacokinetic and statistical analyses are performed on the data obtained from the subjects. The pharmacokinetic parameters are defined as follows:
- AUCₜ₁₋ₜ₂:: The area under the serum concentration versus time curve, from time t1 (in hours) to time t2 (in hours) measurable concentration of the administered drug, as calculated by the linear trapezoidal method. For e.g. AUCₜ₀₋ₜ₂₄ refers to the area under the serum concentration versus time curve, from time 0 (zero) hours to time 24 hours post-administration of dose.
- Cₘₐₓ:: The maximum measured serum concentration of the administered drug.
- C_{avg t1-t2}:: The average serum concentration of testosterone obtained by dividing the AUCₜ₁₋ₜ₂/ |t2-t1|, where in t is time post-administration of dose expressed in hours
- Tₘₐₓ:: The time (in hours) at which the maximum measured plasma concentration of the administered drug is achieved
- Mean:: Average value of measured parameter of all individual subjects.
- C_{avg t0-t24}:: The average serum concentration of testosterone obtained by dividing the AUC t₀₋ₜ₂₄ value by 24. This represents the average serum testosterone level over a period starting from time 0 (zero) hours to time 24 hours post- administration of dose. It should also be noted that C_{avg t0-t24} is also referred to as simply "C_{avg}"
- C_{avg t0-t12}:: The average serum concentration of testosterone obtained by dividing the AUC t₀₋ₜ₁₂ value by 12. This represents the average serum testosterone level over a period starting from time 0 (zero) hours to time 24 hours post -administration of dose.
- C_{avg t12-t24}: : The average serum concentration of testosterone obtained by dividing the AUC t₁₂₋ₜ₂₄ value by 12. This represents the average serum testosterone level over the second half of the 24-hours post-administration of dose period; i. e from a period starting from time 12 hours to time 24 hours post-administration of dose.
Some of the pharmacokinetic results for the compositions indicated therein, are summarized in Tables below.

### EXAMPLE 3 - Comparative testosterone ester compositions

Comparative testosterone ester compositions are prepared having testosterone esters shown in Table 2. The compositions are prepared as described in Example 4 below and tested according to pharmacokinetic (PK) procedure described in Example 2. The PK results following a single dose oral administration of each of Compositions 3-7 with a meal are summarized in Tables 2A, 2B and 2C.

**Table 2:**

| | **Composition (weight %)** | | | | |
|---|---|---|---|---|---|
| **Composition No.** | **3** | **4** | **5** | **6** | **7** |
| Component | | | | | |
| Testosterone undecanoate (T11) | 12 - 20% | - | - | - | - |
| Testosterone dodecanoate (T12) | - | 12 - 20% | - | - | - |
| Testosterone tridecoate (T13) | - | - | 12 - 20% | - | - |
| Testosterone tetradecoate (T14) | - | - | - | 12 - 20% | - |
| Testosterone palmitate (T16) | - | - | - | - | 12 - 20% |
| Lipophilic additive (e.g. Lipophilic surfactant) | 55 - 70 % | 55 - 70 % | 55 - 70 % | 55 - 70 % | 55 - 70 % |
| Hydrophilic additive (e.g. Hydrophilic surfactant) | 12 - 20 % | 12 - 20 % | 12 - 20 % | 12 - 20 % | 12 - 20% |
| Adjuvant | q.s. | q.s. | q.s. | q.s. | q.s. |

**Table 2A: Comparative serum testosterone (T) pharmacokinetic results**

| | **Serum T pharmacokinetic results** | | | | | | |
|---|---|---|---|---|---|---|---|
| **Composition No.** | **1A, 1B, 1C** | **2A, 2B, 2C** | **3** | **4** | **5** | **6** | **7** |
| **PK parameter [units]** | | | | | | | |
| Range of mean Cₘₐₓ / mg of T equivalent dose, [ng/dL/mg] | 2.9 - 4.5 | 1.4 - 2.8 | 6.0 - 20.0 | 5 - 18 | 3.2 - 4.1 | 1.6 - 2.6 | 0.8 - 1.3 |
| Range of mean C_{avg t0-t24} / mg of T equivalent dose, [ng/dL/mg] | 1.5 - 2.2 | 1.2 - 1.8 | 2.8 - 3.5 | 2.4 - 2.6 | 1.7 - 2.1 | 1.4 - 1.7 | <1 |
| Range of the mean C_{avg t12 t24} as % of the mean C_{avg t0-t24h} | 35-70 | 35-70 | 25-34 | 27-33 | 40 - 60 | 38 - 62 | >70 |
| Duration of post-dosing time with serum T at > 300 ng/dL (Hours) | 12 -20 | 12 -20 | 8 -10 | 8 -11 | 14 - 18 | 13 - 18 | < 4 |
| Ratio of mean C_{avgt0-t12} to mean C_{avg t12-t24} | 1 : 07 - 1 : 1.5 | 1 : 0.7 - 1: 1.5 | 1: 0.4 | 1:0.6 | 1: 1.1 | 1: 0.8 | -- |
| Mean AUC_{inf}/ mg T equivalent dose, ng*h/dL | 43.3 - 59.0 | 43.3 - 59.0 | 80.1 | 59.9 | 45.8 - 58.5 | 44.2 - 56.5 | < 30 |

The compositions of Table 2 can be formulated as a capsule or tablet dosage form. Further, each of the dosage form can be formulated to contain from about 100 mg to about 400 mg of the total ester. For instance, the compositions of Table 2 used for the pharmacokinetic studies have been formulated as a capsule dosage form prepared similarly as described under Example 4. The total daily testosterone ester dose administered is 420 to 1250 mg for compositions in Tables 2. Specifically, compositions 1A, 1B, 1C and 5 are administered such that a total daily dose of testosterone tridecoate is from about 420 mg to about 850 mg. Similarly, compositions 2A, 2B, 2C and 6 are administered such that a total daily dose of a total daily dose of testosterone tetradecoate from about 525mg to about 1250 mg.

Composition 7 contains testosterone palmitate and shows poor bioavailability while compositions 3 and 4 containing T11 and T12 testosterone esters respectively are more bioavailable compared to composition 7 and the compositions with T13 testosterone ester (1A, 1B, 1C and 5) and with T14 testosterone ester (2A, 2B, 2C and 6). It is notable that the compositions with T13 and T14 testosterone esters are bioavailable to enable the desired serum testosterone mean C_{avg t0-t24} per mg T equivalent dosed that ranges from 1.2 ng/dL/mg to about 2.2 ng/dL/mg. Further, unlike the Compositions 3 and 4 which provide the serum T levels at > 300 ng/dL for duration of 8-11 hours, the compositions having T13 and T14 testosterone esters sustain the serum T levels at> 300 ng/dL for significantly longer durations (12-20 hours). As can be evidenced from the Table 2A, composition 7, with the much longer chain testosterone ester (T-palmitate), offers significantly less duration of eugonadal serum T levels at a practical daily dose of 350 mg T equivalent. This is probably due to its very high lipophilicity and very low bioavailability.

Further, compositions 3 and 4 (with T11 and T12 testosterone esters, respectively) are more bioavailable relative to composition 7, but the enhanced bioavailability potentially provides for higher Cₘₐₓ values and other disadvantages. The compositions with T13 and T14 testosterone esters are adequately bioavailable to enable desired serum testosterone mean C_{avg t0-t24} per mg T equivalent dose that ranges from about 1.2 ng/dL/mg to about 2.2 ng/dL/mg and mean Cₘₐₓ per mg T equivalent dose that ranges from about 1.4 of 4.5 ng/dL/mg. Such profiles enable patient-friendly dosing regimen (lower total daily T equivalent dose, less frequent administration in a 24 hours period and with fewer number of dosage units per dosing).

Further, as can be seen from the Table 2A, in contrast to the Compositions 3 and 4 (with low lipophilicity esters T11 and T12, respectively), the compositions 1A, 1B, 1C, 5, 2A, 2B, 2C and 6 with lipobalanced esters T13 or T14, are adequately bioavailable and provide longer-lasting serum T levels enabling the desired serum T C_{avg t0-t12}/C_{avg t12-t24} ratio to be between 1:0.7 to 1 :1.5 and also enable serum T levels such that the C_{avg t12-t24} is within 35% to 70% of the C_{avg t0-t24}.

Table 2B below shows the comparative simulations after two consecutive administrations- twice daily (about every twelve hours) for 24 hours or once daily for 48 hours, of Compositions 3-7 with meals to subjects. Specifically, Table 2B shows the duration (in a 24-hour period) during which the serum testosterone for a subject fell below 300 ng/dL.

**Table 2B: Serum testosterone PK parameters after BID administration of Compositions 3-7**

| **Composition No.** | **Time (in hours) below 300 ng/dL in a 24 hour period** | |
|---|---|---|
| | Once-a-day administration | Twice a day administration |
| 3 | 12-14 | 8-9 |
| 4 | 9 - 12 | 8-9 |
| 5 | 5 - 7 | 1- 3.4 |
| 6 | 3 - 7 | 0.5 - 3.4 |

Similarly, Table 2C shows the comparative steady state simulations (attained after daily administration for at least 7 days) for serum testosterone PK parameters for compositions 3 through 7 administered once or twice daily with meals to each subject in a group of at least 12 subjects.

**Table 2C: Steady state serum testosterone PK parameters following daily administration of compositions 3-7**

| **Composition no.** | **Once daily administration** (e.g. 24 h apart) | | **Twice daily administration** (e.g. 12 h apart) | |
|---|---|---|---|---|
| | Mean time with T conc. <300 ng/dL | % of patients in a group with serum T < 300 ng/dL for >7.0 h | Mean time with T conc. < 300 ng/dL | % of patients in a group with T conc. <300 ng/dL for >3.5 h |
| 3 | 8-14h | >80 | 4.0-7.5 h | >50 |
| 4 | 8-10 h | >70 | 3-5.5 h | >50 |
| 5 | 4-7h | <50 | 0.5-3.5 h | <20 |
| 6 | 3.5-7h | <40 | 0.3-2.2 h | <20 |

It is apparent from the pharmacokinetic results in Tables 2B and 2C that compositions 5 (with T13 testosterone ester) and 6 (with T14 testosterone ester) offer distinctive advantages over the compositions 3 and 4 (with low lipophilic testosterone esters T11 and T12, respectively) and are unique with respect to maintaining a majority of the patients (% of patients in a group) that do not slip into hypogonadal levels (<300 ng/dL) for more than 7 hours duration in a 24-hour period, when administered once daily for at least 7 days. Similarly, the inventive compositions with T13 and T14 testosterone esters are unique with respect to maintaining a majority of the patients (% of patients in a group) that do not slip into hypogonadal levels (<300 ng/dL) for more than 3.5h in a 24-hour period, when administered twice daily for at least 7 days.

**Table 2D: Unique dose ranges of the inventive compositions of T13 and T14**

| **Composition No.** | **Dose or dose range as mg T equivalent** | **Serum T pharmacokinetics** | | |
|---|---|---|---|---|
| | | **C_{avg} 0-24h ng/dL** | **C_{avg} 12-24h ng/dL** | **Cₘₐₓ ng/dL** |
| 5 | 220 | <300 | <300 | <1500 |
| | 250-600 | >300 | >300 | <1500 |
| | 750 | >300 | >300- | >1500 |
| 6 | 220 | <300 | <300 | <1500 |
| | 300-700 | >300 | >300 | <1500 |
| | 750 | >300 | >300 | >1500 |
| 3 | 350-750 | >300 | <300 | >1500 |
| | 200-340 | >300 | <300 | >1500 |
| 7 | 250 | <300 | >300 | <1500 |

As shown in Table 2D, unlike compositions of non-lipobalanced testosterone esters (e.g. T11 and T16), the compositions of the unique lipobalanced T13 and T14 testosterone esters are useful for treatment of hypogonadism in the daily dose range of about 250 mg to 700 mg T equivalent for sustained action. In other words, the unique lipobalanced T13 and T14 testosterone esters are useful for treatment of hypogonadism in the said daily dose range of about 420 mg to about 1250 mg of the ester.

**Table 2E: Testosterone pharmacokinetic results summary**

| **Composition No.** | **Range of mean T C_{avg t0-t24} per mg T [ng/dL/mg]** | **Range of Mean T Cₘₐₓ per mg T fng/dL/mg]** |
|---|---|---|
| 3 | 2.8 to 3.5 | 6.0 to 20.0 |
| 4 | 2.4 to 2.6 | 5 to 18 |
| 5 | 1.5 to 2.2 | 2.9 to 4.5 |
| 6 | 1.2 to 1.8 | 1.4 to 2.8 |
| 7 | <1 | 0.8 to 1.3 |

Table 2E shows that unlike composition 7 (T16 testosterone ester), which is poorly bioavailable with lower serum Cₘₐₓ, compositions 3 (T11 testosterone ester) and 4 (T12 testosterone ester), which provide faster rate of T appearance in serum with higher Cₘₐₓ, the compositions 5 and 6 with T13 and T14 testosterone ester, respectively, are adequately bioavailable with a rate of T appearance in the serum leading to the desirable much safer mean Cₘₐₓ per mg T equivalent dose ranging between 1.4 ng/dL/mg to 4.5 ng/dL/mg.

### EXAMPLE 4 -T13 and T14 testosterone ester-containing compositions

Example compositions including T13 and T14 testosterone esters were prepared in accordance with the components set forth in Tables 4 and 5.

**Table 4: Compositions for use according to the invention**

| | **Composition (weight %)** | | | | | |
|---|---|---|---|---|---|---|
| **Composition No.** | **8** | **9** | **10** | **11** | **12** | **13** |
| **Component** | | | | | | |
| T 13 or T14 testosterone ester | 12-20 | 14-26 | 12-20 | 10-30 | 15-22 | 18-26 |
| Lipophilic additive [e.g. Lipophilic surfactant] | 50 - 80 | 55-80 | 55-80 | 50 - 80 | 55 - 70 | 60 - 80 |
| Hydrophilic additive [e.g. Hydrophilic surfactant] | 2 - 30 | < 5 | >20 | 2-20 | 5 - 15 | 2 - 7 |
| Adjuvants | q.s. | q.s | q.s | q.s | q.s | q.s |

The compositions disclosed in Table 4 can be prepared by weighing required amounts of lipophilic additive and hydrophilic additive into a jacketed mixing tank heated to temperature of 70±10°C and gently mixing. The desired amount of drug is added to the container with the molten mixture of the additive under stirring. The admixed compositions are stirred continually until all the drug is uniformly dispersed in the molten additives mixture. The resultant uniform mixture is then filled into gelatin capsules to a predetermined weight, to provide capsule dosage form each containing about 100 mg to 400 mg testosterone ester. It is also notable that the compositions of Table 4 can be formulated in tablet dosage forms. Further, each of the tablet dosage forms can be formulated to contain from about 100 mg to about 1000 mg of the total T13 or T14 ester.

The total daily testosterone ester dose administered is 420 mg to 1250 mg for compositions in Tables 4 formulated as e.g., capsule dosage form. Specifically, when the composition has Testosterone tridecoate, the dose administered is from about 420 mg to about 850 mg. Similarly, when the composition has testosterone tetradecoate, the total daily testosterone ester dose administered is from about 525mg to about 1250 mg. Furthermore, compositions 8 through 13 or dosage form thereof, upon single dose administration to a human subject, can provide a mean serum testosterone C_{avg 12-24h} of 35% to 70% of the C_{avg 0-24h}. While the lipophilic surfactant (e.g. HLB <10) is exemplified as the lipophilic additive in the Compositions 8 to 13, it should be noted that other lipophilic additives such as fatty acid glycerides (monoglyceride, a diglyceride, tocopherol, tocopherol derivatives, or a mixture) and triglycerides and combinations thereof can be used.

Such lipophilic additives can include, but are not limited to, fatty acids (fatty acids of C₆-C₂₂, like oleic, linoleic, palmitic, myristic); triglycerides (like castor oil, corn oil, palm oil, coconut oil, hydrogenated castor oil, soybean oil, etc.); mono-, di-, tri-glycerides or combinations of mono-, di- or tri-glycerides of fatty acids (like glyceryl monooleate [Maisine 35-1®], mono-,di- glycerides of caprylic and capric acid [Capmul MCM®] glyceryl monolinoleate, glyceryl mono laurate, glyceryl distearate, glyceryl monostearate); PG esters of fatty acids (propylene glycol monolaurate, propylene glycol dicaprylate/dicaprate, propylene glycol caprylate/caprate), polyglycerized fatty acids (polyglycerol-3 oleate, polyglyceryl-6 dioleate); alcohol-oil transesterification products (e.g. PEG-6 corn oil, PEG-6 apricot kernel oil, PEG-4 caprylic/capric triglyceride, PEG-20 sorbitan monostearate); alcohol fatty acid esters (isopropyl myristate); sorbitan fatty acid esters (sorbitan monooleate), lipophilic Sterol Surfactants such as cholesterol, sitosterol, phytosterols (e.g. GENEROL series from Henkel), PEG-5 soya sterol (e.g. Nikkol BPS-S, from Nikko), PEG-10 soya sterol (e.g. Nikkol BPS-10 from Nikko), PEG-20 soya sterol (e.g. Nikkol BPS-20 from Nikko).

Similarly, when a hydrophilic additive is present in the composition, it can be a hydrophilic additive or one or more of the hydrophilic surfactant having HLB>10. The optional hydrophilic surfactants can include, but are not limited to, alcohol-oil transesterification products (e.g. PEG-8 caprylic/capric glycerides, lauroyl macrogol-32 glyceride, stearoyl macrogol glyceride); polyoxyethylene hydrogenated vegetable oils (e.g. PEG-40 hydrogenated castor oil); polyoxyethylene vegetable oils (e.g. PEG-35 castor oil); ionic surfactants (e.g. sodium lauryl sulfate, sodium dioctyl sulfosuccinate); polyethylene glycol fatty acids esters; polyethylene glycol fatty acids mono- and di- ester mixtures; and polysorbate 80. Hydrophilic sterol surfactants such as lanosterol PEG-24 cholesterol ether (e.g. Solulan C-24, Amerchol), PEG-30 soya sterol (e.g. Nikkol BPS-30, from Nikko), PEG-25 phyto sterol (e.g. Nikkol BPSH-25 from Nikko), PEG-30 cholestanol (e.g. Nikkol DHC, from Nikko) can also be used as hydrophilic surfactants.

The adjuvants can be pharmaceutical aids and processing aids, fillers, binders flavors, pH modifiers, gelling polymers, pH-sensitive polymers, buffering agents, thickeners, solidifying agents and the like. Few examples include, but not limited to glycerol; propylene glycol; polyethylene glycol (e.g. PEG 300, 6000, 8000 or 20000); aromatic esters (e.g. benzyl benzoate); antioxidants (ascorbyl palmitate, butylated hydroxy anisole, butylated hydroxy toluene, propyl gallate, tocopherol); acids; bases; salts; suffers; amides;; sorbitol; celluloses; cellulose esters; cyclodextrins; silicon dioxides; pyrrolidones; polyvinyl alcohols; sterols and sterol derivatives; tocopherols; tocopherol esters; polyethylene glycol derivatives of tocopherol; silicone oils; simethicones; waxes; shellac; paraffins; and mixtures thereof.

**Table 5: Representative Compositions of the inventive lipobalanced testosterone esters**

| | **Composition (weight %)** | | |
|---|---|---|---|
| **Composition No.** | **14** | **15** | **16** |
| Component | | | |
| T13 or T14, | 12- 30 | 15-25 | 10-22 |
| Lipophilic surfactant (eg. Glyceryl monolinoleate) | 55-80 | 50-80 | 55-80 |
| Hydrophilic surfactant (polyoxyl hydrogenated castor oil) | 0-20 | 0-20 | 0-20 |
| Alcohol (e.g. ethanol) | > 10 | 0 | <10 |
| Triglyceride (e.g. castor oil) | - | - | <50 |
| Adjuvant* | q.s | q.s. | q.s. |

| | | | |
|---|---|---|---|
| *Optional | | | |

The lipophilic additives, the hydrophilic additives and the adjuvant for the representative inventive compositions shown in Table 5, can be similar to that described for compositions in Table 4. It is also notable that the Compositions 14 to 16 can be formulated as a capsule or tablet dosage form. Further, each of the dosage form can be formulated to contain from about 100 mg to about 400 mg of the total ester. For instance, the Compositions 14 to 16 can be formulated as a capsule dosage form prepared as described under Example 4.

Compositions 14 through 16 or dosage form thereof, upon single dose administration to a group of human subjects, can provide a mean serum testosterone C_{avg t12-t24} of 35% to 70% of the mean serum testosterone C_{avg t0-t24}. All of the representative compositions or dosage forms of Tables 4 and 5 can provide upon a single dose administration with meal, a mean serum T C_{avg t0-t24} per mg T equivalent within a range of about 1.2 to 2.2 ng/dL/mg T; a mean serum T Cₘₐₓ per mg T equivalent within a range of about 1.4 to about 1.5 ng/dL/mg T; and a mean serum T C_{avg t12-t24} that is between about 35 to about 70% of the mean serum T C_{avg t0-t24} The total daily testosterone ester dose administered can be from about 420 to about 1250 mg. Specifically, when the composition has testosterone tridecoate, the dose administered is from about 420 mg to about 850 mg. Similarly, when the composition has testosterone tetradecoate, the total daily testosterone ester dose administered is from about 525mg to about 1250 mg.

### EXAMPLE 5 -Clinical Trial

The following is an overview of the early phase clinical trial design for the compositions of the unique T13 and T14 testosterone esters of the current invention.

Subject Population: Group of Men of age 18-65 years of age with morning serum testosterone levels <300 ng/dL at two measurements, at least 1 hour apart

### Treatment Groups

- Treatment A:: Dosage Form of composition 5 administered at a starting dose of 250 mg T equivalent once daily with meal
- Treatment B:: Dosage Form of composition 6 administered at starting dose of 325 mg T equivalent once daily with meal
- Treatment C:: Dosage Form of composition 5 administered at a starting dose of 150 mg T equivalent twice daily (12 hours apart, total daily dose of 300 mg T equivalent) with meal
- Treatment D:: Dosage Form of composition 6 administered at a starting dose of 175 mg T equivalent twice daily (12 hours apart, total daily dose of 350 mg T equivalent) with meal
- Treatment E:: Dosage Form of composition 3 administered at a starting dose of 250 mg T equivalent once daily with meal
- Treatment F:: Dosage Form of composition 7 administered at a starting dose of 450 mg T equivalent once daily with meal

At three and/or seven weeks after dosing, based on the concentration of serum testosterone, the daily testosterone ester dose may be titrated by upward or downward dose adjustment, by up to 50% of the initial or the previous daily dose. On day 84, multiple blood samples drawn from the subjects are used for PK parameter determinations.

To assess the performance of different compositions for use according to the invention, the group responder analyses were performed; accordingly, the number of subjects with C_{avg} within 300-1000 ng/dL with a 90% CI and % of subjects with Cₘₐₓ <1500, Cₘₐₓ between 1800-2500 and % of subjects with Cₘₐₓ >2500 ng/dL, estimated. The anticipated results from the study along with criteria for effective and safe testosterone therapy are shown in Table 3 below.

**Table 3: Group responder analysis of PK parameters against desired responder group %**

| **Parameter based on serum T levels** | **Criteria** | **% Responders in Treatment Groups** | | | | | |
|---|---|---|---|---|---|---|---|
| | | **A** | **B** | **C** | **D** | **E** | **F** |
| C_{avg} 300-1140 ng/dL | ≥ 75% | 75-100 | 75-100 | 75-100 | 75-100 | 100 | 62 |
| Cₘₐₓ ≤ 1500 ng/dL | ≥ 85 % | 85-100 | 85-100 | 85-100 | 85-100 | 60 | 100 % |
| 1800 < Cₘₐₓ < 2500 ng/dL | ≤ 5 % | 0-5 | 0-5 | 0-5 | 0-5 | 5-10 | 0-5 |
| Cₘₐₓ > 2500 ng/dL | 0% | 0-2 | 0-2 | 0-2 | 0-2 | 0-2 | 0 |

Based on the above group responder analysis from the pharmacokinetic data for the four treatments, dosage form of Composition 5 with T13 testosterone ester administered at a starting dose of 250 mg T equivalent (420 mg T13 testosterone ester) once daily or at a starting dose of 150 mg T equivalent twice daily, about 12 hours apart (total daily dose of 300 mg T equivalent, or about 505 mg T13 testosterone ester) with meal would enable safe and effective testosterone replacement therapy. Similarly, the dosage form of Composition 6 with T14 testosterone ester administered at a starting dose of 325 mg T equivalent (about 758 mg T14 testosterone ester) once daily or at a starting dose of 175 mg T equivalent twice daily, about 12 hours apart (total daily dose of 350 mg T equivalent or about 625 mg T14 testosterone ester) along with meal would enable safe and effective testosterone replacement therapy. Whereas, for a patient-friendly dosing regimen, Composition 3 fails to meet the acceptable responder criteria for safety (serum T Cₘₐₓ), Composition 7 (with T16 testosterone ester) does not meet the acceptable responder criteria for efficacy (serum T C_{avg}), likely due to low or very high lipophilicity of the esters, respectively.

### EXAMPLE 6 - Solubility of various Testosterone Esters in various lipophilic Additives

The solubility of testosterone esters (undecanoate, dodecanoate, tridecoate and tetradecoate) was determined in various lipophilic additives such as a long chain triglyceride (e.g. castor oil), long chain fatty acid (e.g. oleic acid), and mono-, diglyceride- (e.g. glyceryl mono and di linoleate). The experiment was carried out by shaking added excess individual T ester to the lipophilic additive at room temperature until equilibrium was reached. At equilibrium, the samples were centrifuged and the supernatant analyzed by HPLC using standards of the respective ester. Each testosterone ester's solubility was estimated as mg of the ester dissolved in 1 g of solution. Solubility in oleic acid, glyceryl mono-/di- linoleate, and castor oil as a function of lipophilicty of ester (fatty chain length) is presented in FIGs. 1, 2, and 3 respectively.

It is apparent from the solubility results that testosterone tridecoate (T13) and testosterone tetradecoate (T14) have unexpectedly disproportionate lower solubility given their higher ClogP compared to the Undecanoate (T11) ester. Accordingly, achieving a dosage form with fully dissolved drug with T13 and T14 would require significant number of dosage units in order to provide an adequate dose for therapeutic effectiveness. Lower solubility of the unique T13 and T14 testosterone esters presents difficulties in formulating high drug load fully dissolved compositions with these esters for T therapy.

### EXAMPLE 7 - Unique effective dose range of T13 and T14 Ester-containing compositions

Table 6 shows the expected effects of daily dose (as mg T equivalent) and dosing regimen of compositions of containing T13 and T14 testosterone esters of testosterone on a group responder analysis estimated from the PK results discussed earlier.

**Table 6: Comparative dose effects of dosage forms of T13 and T14 testosterone ester following at least 90 days of treatment**

| **T -Ester (Composition No.)** | **Starting Total mg T Equivalent Dose (± dose adjustment in mg T equivalent)*** | **Dosing Frequency** | **% Responders with C_{ave t0-t24} (ng/dL)** | **% Responders with Cₘₐₓ (ng/dL)** | | |
|---|---|---|---|---|---|---|
| | | | **300-1140** | **≤ 1500** | **1800-2500** | **> 2500** |
| T13 (Composition 5) | 300 (± 50) | QD | 100 | 100 | 0-5 | 0-1 |
| | 350 (± 100) | BID | 100 | 100 | 0-5 | 0-1 |
| | 1000 (± 200) | QD or BID | 100 | 0 | 20-30 | 60-80 |
| | 100 (± 50) | QD or BID | 50-65 | 100 | 0-5 | 0-1 |
| T14 (Composition 6) | 325 (± 75) | QD | 100 | 100 | 0-5 | 0-1 |
| | 375 (± 125) | BID | 100 | 100 | 0-5 | 0 |
| | 1200 (± 200) | QD or BID | 100 | 0 | 15-30 | 60-85 |
| | 100 (± 50) | QD or BID | 30 - 50 | 100 | 0-5 | 0-1 |

| | | | | | | |
|---|---|---|---|---|---|---|
| *(+) for upward or (-) for downward dose titration QD = once-a-day (about every 24 hours) BID = Twice a day (about every 12 hours) | | | | | | |

Based on the above group responder analysis of the pharmacokinetic data, dosage forms containing compositions with T13 ester at daily starting dose of 250- 510-mg T equivalent with a standard American diet meal would enable successful safe and effective testosterone replacement therapy. Similarly, dosage form of the compositions with T14 ester at daily starting dose of 300 mg to 750 mg T equivalent with a standard American diet meal would enable successful safe and effective testosterone replacement therapy. The total daily T13 or T14 testosterone ester dose range administered is 420 to 1250 mg for Compositions 5 and 6. Specifically, the total daily dose of testosterone tridecoate is from about 420 mg to about 850 mg and the total daily dose of testosterone tetradecoate is from about 525 mg to about 1250 mg

### EXAMPLE 8- T13 and T14-containing compositions as capsule dosage forms

**Table 7:**

| | **Composition (weight %)** | | | | | |
|---|---|---|---|---|---|---|
| **Composition No.** | **17** | **18** | **19** | **20** | **21** | **22** |
| Components | | | | | | |
| Testosterone tridecoate | 10 - 30 | 10- 30 | | | 10-30 | |
| Testosterone tetradecoate | - | | 10- 30 | 10 - 30 | - | 10-30 |
| Lipophilic additive [e.g. surfactant of HLB <10 such as. mono- or di- or tri- glyceride of fatty acid] | 55-80 | 55-80 | 50-80 | 55-80 | 55-80 | 55-80 |
| Hydrophilic additive (e.g. Surfactant with HLB >10 such as cremophor RH40) | 0-20 | 0-20 | 0-20 | 0-20 | 0-20 | 0-20 |
| Adjuvant | q.s. | q.s | q.s. | q.s | q.s. | q.s |

| | **Serum T pharmacokinetic results** | | | | | |
|---|---|---|---|---|---|---|
| PK parameter | | | | | | |
| Daily dose as mg T Equivalent | 250-400 | 250-500 | 300-500 | 300-500 | 250-400 | 300-500 |
| % of T-ester not dissolved in lipophilic additive at body temperature | 0 | >12 | 0 | >12 | >40 | >20 |
| % of T-ester not dissolved in lipophilic additive at 20°C | 0 | >15 | 0 | >10 | >50 | >25 |
| No. of capsules /daily T dose | 4-5 | 3-7 | 5-6 | 3-6 | 1-3 | 2-4 |
| Mean serum T C_{avg t0-t24} / mg T equivalent [ng/dL/mg] | 1.65 | 1-2-2.2 | 1.34 | 1.2-2.2 | 1.86 | 1.52 |

It is also notable that Compositions 17 to 22 can be formulated as a capsule or tablet dosage form. Further, each of the capsule dosage forms can be formulated to contain from about 100 mg to about 400 mg of the ester. For instance, the Compositions 17 to 22 can be formulated as a capsule dosage form prepared as described under Example 4.
Total daily ester dose administered is 420 to 1250 mg for Compositions 17-22. Specifically, for Compositions 17, 18 and 21 the total daily T13 testosterone ester dose administered is from about 420 mg to about 850. However, it is notable that unlike Composition 17 that has no "not dissolved" ester, Compositions 18 and 21 require fewer dosage units per administration. Further, for Compositions 19, 20 and 22 the total daily T14 testosterone ester dose administered is from about 525 mg to about 1250 mg. However, it is notable that unlike Composition 19 that has no "not dissolved" ester, Compositions 18 and 21 require fewer dosage units per administration.

Table 7 shows that the higher the fraction of the lipobalanced ester not dissolved or not solubilized, the fewer the number of daily dosage form units (e.g. capsules) that need to be administered to achieve the desirable serum testosterone levels when treating hypogonadism in a male with T13 and T14 testosterone esters. It should be noted that to provide the total daily dose of about 420 mg - 850 mg of the T13 testosterone ester for a hypogonadal subject, no more than four oral dosage form units are required; even more preferred is that no more than two oral dosage form units per day are required for administration. Similarly, to provide the total daily dose of about 525 mg - 1250 mg of the T14 testosterone ester for a hypogonadal subject, no more than six oral dosage form units are required; even more preferred is that no more than three oral dosage form units per day are required for administration.

Compositions 18 to 22 can be prepared with the lipophilic surfactant and hydrophilic surfactant in amounts such that the ratio of amount (wt%) of lipophilic surfactant to amount (wt%) of hydrophilic surfactant is greater than 2:1. Specifically, the ratio of amount (wt%) of lipophilic surfactant to amount (wt%) of hydrophilic surfactant can be greater than 2.5:1. Further, the ratio of amount (wt%) of lipophilic surfactant to amount (wt%) of hydrophilic surfactant can be greater than 3.5:1. Even further, the ratio of amount (wt%) of lipophilic surfactant to amount (wt%) of hydrophilic surfactant can be greater than 6.5:1.

Compositions 18 to 22 can be prepared with hydrophilic surfactant present at 20 wt% or more of the total carrier. Compositions 18 to 22 can be prepared with hydrophilic surfactant present at 5 wt% or less of the total carrier. The lipophilic additives, the hydrophilic additives, and the adjuvant for the representative inventive compositions shown in Table 7 can be similar to those described for compositions in Table 4. The pharmacokinetic (PK) evaluation procedure is given under Example 2. The PK results for the Compositions 18, 20-22 or related capsule dosage forms thereof, following oral administration of single dose, two consecutive doses or steady state to a group of subjects, for example, hypogonadal males, along with a meal, are summarized in Table 7A.

**Table 7A: Serum T pharmacokinetics for Compositions 18 and 20-22 following single administration**

| **PK parameter** | **Results** |
|---|---|
| Range of mean Cₘₐₓ / mg of T equivalent dose, [ng/dL/mg] | 1.4 - 4.5 |
| Range of mean C_{avg t0-t24} / mg of T equivalent dose, [ng/dL/mg] | 1.2 - 2.2 |
| Range of the C_{avg t12-t24} as % of the C_{avg t0-t24} | 35-70 |
| Duration of post-dosing time with serum T at > 300 ng/dL | 12 to 24 hours |

**Table 7B: Serum T pharmacokinetics for Compositions 18 and 20-22 following two consecutive dose administration**

| **PK parameter** | **Results** |
|---|---|
| Time of T concentration below 300 ng/dL following two consecutive administrations 24 hours apart (once daily) within 48 hour time period | 2 to 7 hours |
| Time of T concentration below 300 ng/dL following two consecutive administrations about 12 hours apart (twice daily) within 24 hours | 0.5 to 3.5 hours |

**Table 7C: Steady state serum T pharmacokinetics for Compositions 18 and 20-22 following at least 7 days continuous administration to a group of at least 12 subjects**

| **PK parameter** | **Results** |
|---|---|
| Time of T concentration below 300 ng/dL following once daily administration | 3.5 - 6.5 hours |
| % of patients with serum T < 300 ng/dL for more than 7 hours following once daily administration | < 50 % |
| Time of T concentration below 300 ng/dL following twice daily administration | 0.3 to 3.5 hours |
| % of patients with serum T < 300 ng/dL for more than 7 hours following twice daily administration | < 20 % |

It is noteworthy that unlike Compositions 17 and 19 the testosterone ester in Compositions 18, 20, 21 and 22 is not fully dissolved nor solubilized in the composition or dosage form thereof. Further, Compositions 18, 20, 21, and 22 provide, upon single administration with a meal to a human subject, a serum T mean C_{avg t0-t24} /mg of T equivalent dose administered in a range between the 1.2 to 2.2 ng/dL/mg. Additionally, Compositions 18, 20, 21 and 22 enable a patient-friendly dosing regimen, for instance *via* fewer dosage units per administration.

### EXAMPLE 9 - Additional T13 and T14-Containing Compositions

In some specific embodiments, the inventive compositions can further include another testosterone ester. Formulations including additional testosterone esters can be found in Tables 8 and 8A.

**Table 8:**

| | **Composition (weight %)** | | | | | |
|---|---|---|---|---|---|---|
| **Composition No.** | **23** | **24** | **25** | **26** | **27** | **28** |
| Component | | | | | | |
| Total Testosterone Ester | 10- 50 | | | | | |
| Testosterone Tridecoate | 10-30 % | | | | | |
| Testosterone Tetradecoate | 10 - 30 % | | | | | |
| Testosterone Tridecoate & Tetradecoate | 10-30 % | | | | | |
| Testosterone | | | | | 5-50 % | 5-50 % |
| Testosterone Cypionate | | | 5-50 % | | | |
| Testosterone Propionate | 5-50 % | | | | | |
| Testosterone Phenylpropionate | 5-50 % | | | | | |
| Testosterone Isocaprate | 5-50 % | | | | | 5-50 % |
| Testosterone Decanoate | | | 5-50 % | | | |
| Testosterone Undecanoate | | | | 5-50 % | | |
| Testosterone Dodecanoate | | | | | | |
| Pharmaceutical Carrier (see Table 7B) | q.s. | q.s. | q.s. | q.s. | q.s. | q.s. |

**Table 8A: Carrier components for the compositions 23-28 of Table 8**

| | **Carrier component (weight %)** | | |
|---|---|---|---|
| **Composition No.** | **A** | **B** | **C** |
| Carrier Component | | | |
| Lipophilic Additive | 70-100 % | - | 20-80 % |
| Hydrophilic Additive | - | 70-100 % | 20-80 % |
| Adjuvant | 0-30% | 0-30% | 0-40% |

The compositions disclosed in Table 9 are prepared as described in Example 4, and the capsule fill composition is provided based on an 800 mg weight per dosage form such as capsule.

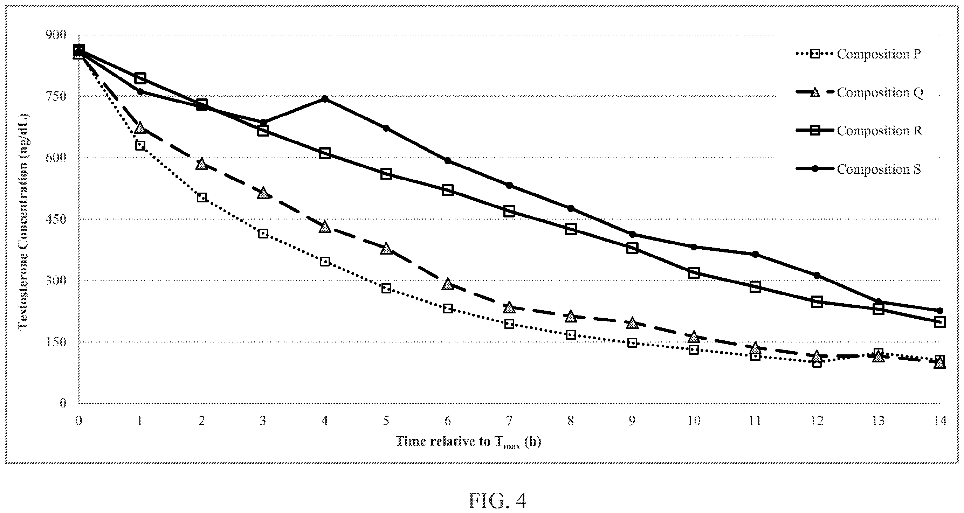

The compositions P, Q, R and S were administered to subjects with meal and tested according to pharmacokinetic (PK) procedure described in Example 2. The data obtained from the PK study for each of the four esters were normalized for Cₘₐₓ and Tₘₐₓ and the PK profile is shown in FIG. 4.

**Table 9: Additional exemplary compositions for use according to the invention**

| **COMPOSITION, mg (weight %)** | | | | | | | | | | | | | **Ratio of LS:HS*** |
|---|---|---|---|---|---|---|---|---|---|---|---|---|---|
| **No.** | **T Ester** | **Drug** | **Oleic Acid** | **Castor Oil** | **Capmul MCM** | **Maisine** | **GDS** | **Alcohol** | **Cremophor RH40** | **Labrasol** | **Tween 80** | **Adjuvant** | |
| A | **T13** | 160 (20%) | - | - | 410 (51%) | - | - | - | 150 (19%) | - | - | 80 (10%) | 2.7:1 |
| B | **T14** | 160 (20%) | - | - | 410 (51%) | - | - | - | 150 (19%) | - | - | 80 (10%) | 2.7:1 |
| C | **T13** | 160 (20%) | - | - | 512 (64%) | - | - | - | - | - | 80 (10%) | 48 (6%) | 6.4:1 |
| D | **T14** | 144 (18%) | - | - | 528 (66%) | - | - | - | - | - | 80 (10%) | 48 (6%) | 6.6 :1 |
| E | **T13** | 160 (20%) | - | 288 (36%) | - | 240 (30%) | - | - | 80 (10%) | - | - | 32 (4%) | 6.6 :1 |
| F | **T14** | 160 (20%) | - | 288 (36%) | - | 240 (30%) | - | - | 80 (10%) | - | - | 32 (4%) | 6.6 :1 |
| G | **T13** | 200 (25%) | - | - | - | 600 (75%) | - | - | - | - | - | - | |
| H | **T14** | 200 (25) | - | - | - | 540 (67.5) | 60 (7.5) | - | - | - | - | - | |
| I | **T13 + T14 (1:1)** | 200 (25) | - | - | - | 540 (67.5) | 60 (7.5) | - | - | - | - | - | |
| J | **T13 + T14 (1:1)** | 240 (30%) | - | - | - | - | - | - | 360 (45%) | 200 (25%) | - | - | |
| K | **T13 + T14 (1:1)** | 240 (30%) | - | - | 400 (50%) | - | - | - | 160 (20%) | - | - | - | 2.5 : 1 |
| L | **T13** | 280 (35%) | 80 (10%) | - | - | 400 (50%) | - | - | 40 (5%) | - | - | - | 12 :1 |
| M | **T14** | 280 (35%) | 80 (10%) | - | - | 400 (50%) | - | - | 80 (10%) | - | - | - | 12:1 |
| N | **T13** | 160 (20%) | - | - | 440 (55%) | - | - | - | 200 (25%) | - | - | - | 2.2:1 |
| O | **T14** | 160 (20%) | - | - | 440 (55%) | - | - | - | 200 (25%) | - | - | - | 2.2:1 |
| P | **T13** | 120 (15%) | - | - | - | 512 (64%) | - | - | 128 (16%) | - | - | 40 (5%) | 4: 1 |
| Q | **T14** | 120 (15%) | - | - | - | 512 (64%) | - | - | 128 (16%) | - | - | 40 (5%) | 4:1 |
| R | **T11** | 120 (15%) | - | - | - | 512 (64%) | - | - | 128 (16%) | - | - | 40 (5%) | 4:1 |
| S | **T12** | 120 (15%) | - | - | - | 512 (64%) | - | - | 128 (16%) | - | - | 40 (5%) | 4:1 |
| T | **T13** | 160 (20%) | - | - | 520 (65%) | - | - | - | 80 (10%) | - | - | 40 (5%) | 6:1 |
| U | **T14** | 160 (20%) | - | - | 520 (65%) | - | - | - | 80 (10%) | - | - | 40 (5%) | 6.5:1 |
| V | **T13** | 120 (15%) | - | - | 500 (62.5%) | - | - | - | - | 132 (16.5%) | - | 48 (6%) | 3.8:1 |
| w | **T14** | 120 (15%) | - | - | 500 (62.5%) | - | - | - | - | 132 (16.5%) | - | 48 (6%) | 3.8:1 |
| X | **T13** | 144 (23.2%) | - | - | 440 (71%) | - | - | - | - | - | 20 (3.2%) | 16 (2.6%) | 22:1 |
| Y | **T14** | 144 (23.2%) | - | - | 440 (71%) | - | - | - | - | - | 20 (3.2%) | 16 (2.6%) | 22:1 |
| Z | **T13** | 140 (17.5%) | - | - | 240 (30%) | 224 (28%) | - | - | 72 (9%) | 72 (9%) | - | 52 (6.5%) | 3.2:1 |
| AA | **T14** | 140 (17.5%) | - | - | 240 (30%) | 224 (28%) | - | - | 72 (9%) | 72 (9%) | - | 52 (6.5%) | 3.2:1 |
| AB | **T13** | 140 (17.5%) | - | - | 200 (25%) | 212 (26.5%) | - | 64 (8%) | 68 (8.5%) | 64 (8%) | - | 52 (6.5%) | 3.1:1 |
| AC | **T14** | 140 (17.5%) | - | - | 200 (25%) | 212 (26.5%) | - | 64 (8%) | 68 (8.5%) | 64 (8%) | - | 52 (6.5%) | 3.1:1 |
| AD | **T13** | 140 (17.5%) | - | - | 200 (25%) | 212 (26.5%) | - | 24 (3%) | 80 (10%) | 80 (10%) | - | 64 (8%) | 2.6:1 |
| AE | **T14** | 140 (17.5%) | - | - | 200 (25%) | 212 (26.5%) | - | 24 (3%) | 80 (10%) | 80 (10%) | - | 64 (8%) | 2.6 :1 |
| AF | **T13** | 360 (39%) | - | - | - | 450 (49) | - | - | 60 (7) | - | - | 50 (5) | 7.5:1 |
| AG | **T14** | 360 (39%) | - | - | - | 450 (49) | - | - | 60 (7) | - | - | 50 (5) | 7.5:1 |

| | | | | | | | | | | | | | |
|---|---|---|---|---|---|---|---|---|---|---|---|---|---|
| *LS= Lipophilic surfactant; HS=Hydrophilic surfactant | | | | | | | | | | | | | |

**Table 10:**

| | **Composition (weight %)** | | | | | | | |
|---|---|---|---|---|---|---|---|---|
| **Composition No.** | **29** | **30** | **31** | **32** | **33** | **34** | **35** | **36** |
| **Component** | | | | | | | | |
| T 13 testosterone ester | 12-30 | 15 | 12-30 | 15 | - | - | - | - |
| T14 testosterone ester | - | - | - | - | 12-30 | 15 | 12-30 | 15 |
| Lipophilic additive [e.g. lipophilic surfactant such as Maisine 35-1, Capmul MCM, etc.] | 20-80 | 40 | - | - | 20-80 | 40 | - | - |
| Lipophilic additive [e.g. lipophilic phytosterol surfactant such as cholesterol, sitosterol, Generol®, PEG-5 soya sterol, PEG-10 soya sterol, PEG-20 soya sterol...] | 20-80 | 24 | 50-80 | 70 | 20-80 | 24 | 50-80 | 70 |
| Hydrophilic additive [e.g. hydrophilic surfactant like Cremophor RH 40, polysorbate 80] | 0-30 | 6 | - | - | 0-30 | 6 | - | - |
| Hydrophilic additive [e.g. hydrophilic phytosterol surfactant like lanosterol PEG-24 cholesterol ether, PEG-30 soya sterol, PEG-25 phyto sterol, | 0-30 | 10 | 5-40 | 10 | 0-30 | 10 | 5-40 | 10 |
| PEG-30 cholestanol, etc.] | | | | | | | | |
| Adjuvants [e.g. PEG 8000, etc.] | q.s. | 5 | q.s | 5 | q.s. | 5 | q.s | 5 |
| Ratio of LS:HS* | - | 4:1 | - | 7:1 | - | 4:1 | - | 7:1 |

| | | | | | | | | |
|---|---|---|---|---|---|---|---|---|
| *LS= Lipophilic surfactant; HS=Hydrophilic surfactant | | | | | | | | |

The compositions disclosed in Table 10 have phytosterols as lipophilic and/or hydrophilic surfactants and are prepared as described in Example 4. The compositions 30, 32, 34 and 36 can be made into a dosage form like a capsule or a tablet.

It is understood that the above-described various types of compositions, dosage forms and/or modes of applications are only illustrative of preferred embodiments of the present invention. Numerous modifications and alternative arrangements may be devised by those skilled in the art without departing from the scope of the present invention and the appended claims are intended to cover such modifications and arrangements. Thus, while the present invention has been described above with particularity and detail in connection with what is presently deemed to be the most practical and preferred embodiments of the invention, it will be apparent to those of ordinary skill in the art that variations including, but not limited to, variations in size, materials, shape, form, function and manner of operation, assembly and use may be made without departing from the principles and concepts set forth herein.

## Claims

1. An oral pharmaceutical composition for use in the treatment of a condition associated with testosterone deficiency in a human subject, the composition comprising:
a testosterone ester having a structure: 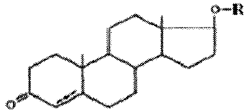
wherein R is -C₁₃H₂₅O or -C₁₄H₂₇O, and one or both esters can be present in the composition; and
a pharmaceutically acceptable carrier,
wherein the composition is formulated as an oral dosage form,
wherein the treatment comprises administering to the human subject the oral dosage form to provide a daily testosterone ester dose of about 420 mg to about 1250 mg based on single unit or multiple unit dosing, and
wherein the human subject is a hypogonadal male.

2. The oral pharmaceutical composition for use according to claim 1 wherein, upon single administration to a group of hypogonadal males, the composition is capable of providing a mean serum testosterone C_{avg 12-24h} that is within about 35% to about 70% of the mean serum testosterone C_{avg 0-24h}.

3. The oral pharmaceutical composition for use according to claim 1 or 2, wherein the dosage form is formulated for once-a-day administration.

4. The oral pharmaceutical composition for use according claim 1 or 2, which is based on multiple unit dosing and comprises administering the entire required dose of the testosterone ester per administration time in the form of two or more unit dosage forms.

5. The oral pharmaceutical composition for use of claim 1 or 2, wherein R is -C₁₃H₂₅O and when a daily dose of 420 mg to 850 mg testosterone ester is administered continuous once-a-day to each subject in a group of hypogonadal males for a period of at least 84 days, the administration is such that at least 75% of the hypogonadal males in the group have a serum testosterone C_{avg} of about 300ng/dL to about 1100 ng/dL, and at least one of the following:
a serum testosterone Cₘₐₓ of less than 1500 ng/dL in at least 85% of the subjects in the group;
a serum testosterone Cₘₐₓ of about 1800 ng/dL to about 2500 ng/dL in 10% or less of the subjects in the group; and
a serum testosterone Cₘₐₓ greater than 2500 ng/dL in about 5% or less of the subjects in the group.

6. The oral pharmaceutical composition for use of claim 1 or 2, wherein R is -C₁₄H₂₇O and when a daily dose of 525 mg to 1250 mg testosterone ester is administered continuous once-a-day to each subject in a group of hypogonadal males for a period of at least 84 days, the administration is such that at least 75% of the hypogonadal males in the group have a serum testosterone C_{avg} of about 300ng/dL to about 1100 ng/dL, and at least one of the following:
a serum testosterone Cₘₐₓ of less than 1500 ng/dL in at least 85% of the subjects in the group;
a serum testosterone Cₘₐₓ of about 1800 ng/dL to about 2500 ng/dL in 10% or less of the subjects in the group; and
a serum testosterone Cₘₐₓ greater than 2500 ng/dL in about 5% or less of the subjects in the group.

7. The oral pharmaceutical composition for use of claim 1 or 2, wherein the pharmaceutically acceptable carrier includes a lipophilic additive which is a lipophilic surfactant.

## Patentansprüche

1. Orales Arzneimittel zur Verwendung bei der Behandlung eines Zustands in Zusammenhang mit Testosteronmangel in einem menschlichen Individuum, wobei das Arzneimittel umfasst:
einen Testosteronester mit der Struktur: 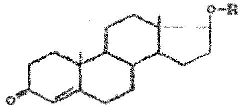
wobei R gleich -C₁₃H₂₅O oder -C₁₄H₂₇O ist und ein oder beide Ester im Arzneimittel vorhanden sein können; und
einen pharmazeutisch verträglichen Träger,
wobei das Arzneimittel als orale Darreichungsform formuliert ist,
wobei die Behandlung das Verabreichen der oralen Darreichungsform an das menschliche Individuum umfasst, um eine tägliche Testosteronesterdosis von etwa 420 mg bis etwa 1250 mg basierend auf Einfach- oder Mehrfachdosierung bereitzustellen, und
wobei das menschliche Individuum ein hypogonadaler Mann ist.

2. Orales Arzneimittel zur Verwendung nach Anspruch 1, wobei das Arzneimittel in der Lage ist, nach einmaliger Verabreichung an eine Gruppe hypogonadaler Männer einen mittleren Serum-Testosteronwert C_{avg 12-24h} bereitzustellen, der zwischen etwa 35% bis etwa 70% des mittleren Serum-Testosteronwerts C_{avg 0-24h} liegt.

3. Orales Arzneimittel zur Verwendung nach Anspruch 1 oder 2, wobei die Darreichungsform für eine Einmal-pro-Tag-Verabreichung formuliert ist.

4. Orales Arzneimittel zur Verwendung nach Anspruch 1 oder 2, das auf Mehrfachdosierung beruht und das Verabreichen der gesamten erforderlichen Dosis an Testosteronester pro Verabreichungszeit in Form von zwei oder mehr Einheitsdosierungsformen umfasst.

5. Orales Arzneimittel zur Verwendung nach Anspruch 1 oder 2, wobei R gleich -C₁₃H₂₅O ist und wenn eine tägliche Dosis von 420 mg bis 850 mg Testosteronester kontinuierlich einmal täglich an jedes Individuum in einer Gruppe hypogonadaler Männer über einen Zeitraum von mindestens 84 Tagen verabreicht wird, die Verabreichung derart ist, dass mindestens 75% der hypogonadalen Männer in der Gruppe einen Serum-Testosteronwert C_{avg} von etwa 300 ng/dl bis etwa 1100 ng/dl haben, und mindestens eines der Folgenden:
einen Serum-Testosteronwert Cₘₐₓ niedriger als 1500 ng/dl bei mindestens 85% der Individuen in der Gruppe;
einen Serum-Testosteronwert Cₘₐₓ von etwa 1800 ng/dl bis etwa 2500 ng/dl bei 10% oder weniger der Individuen in der Gruppe; und
einen Serum-Testosteronwert Cₘₐₓ höher als 2500 ng/dl bei etwa 5% oder weniger der Individuen in der Gruppe.

6. Orales Arzneimittel zur Verwendung nach Anspruch 1 oder 2, wobei R gleich -C₁₄H₂₇O ist und wenn eine tägliche Dosis von 525 mg bis 1250 mg Testosteronester kontinuierlich einmal täglich an jedes Individuum in einer Gruppe hypogonadaler Männer über einen Zeitraum von mindestens 84 Tagen verabreicht wird, die Verabreichung derart ist, dass mindestens 75% der hypogonadalen Männer in der Gruppe einen Serum-Testosteronwert C_{avg} von etwa 300 ng/dl bis etwa 1100 ng/dl haben, und mindestens eines der Folgenden:
einen Serum-Testosteronwert Cₘₐₓ niedriger als 1500 ng/dl bei mindestens 85% der Individuen in der Gruppe;
einen Serum-Testosteronwert Cₘₐₓ von etwa 1800 ng/dl bis etwa 2500 ng/dl bei 10% oder weniger der Individuen in der Gruppe; und
einen Serum-Testosteronwert Cₘₐₓ höher als 2500 ng/dl bei etwa 5% oder weniger der Individuen in der Gruppe.

7. Orales Arzneimittel zur Verwendung nach Anspruch 1 oder 2, wobei der pharmazeutisch verträgliche Träger einen lipophilen Zusatzstoff umfasst, der eine lipophile oberflächenaktive Substanz ist.

## Revendications

1. Composition pharmaceutique orale pour une utilisation dans le traitement d'une affection associée à une déficience en testostérone chez un sujet humain, la composition comprenant :
un ester de testostérone ayant une structure : 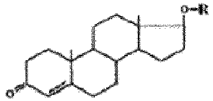
dans laquelle R est -C₁₃H₂₅O ou -C₁₄H₂₇O, et l'un ou les deux esters peuvent être présents dans la composition ; et
un vecteur pharmaceutiquement acceptable,
dans laquelle la composition est formulée en tant que forme galénique orale,
dans laquelle le traitement comprend l'administration au sujet humain de la forme galénique orale pour fournir une dose quotidienne d'ester de testostérone d'environ 420 mg à environ 1 250 mg sur la base d'une administration de doses unitaires uniques ou multiples, et
dans laquelle le sujet humain est un mâle hypogonadique.

2. Composition pharmaceutique orale pour une utilisation selon la revendication 1 dans laquelle, lors d'une administration unique à un groupe de mâles hypogonadiques, la composition est capable de fournir une C_{avg 12-24 h} de testostérone sérique moyenne qui est d'environ 35 % à environ 70 % de la C_{avg 0-24h} de testostérone sérique moyenne.

3. Composition pharmaceutique orale pour une utilisation selon la revendication 1 ou 2, dans laquelle la forme galénique est formulée pour une administration une fois par jour.

4. Composition pharmaceutique orale pour une utilisation selon la revendication 1 ou 2, qui est basée sur une administration de doses unitaires multiples et comprend l'administration de la dose requise entière de l'ester de testostérone par temps d'administration sous la forme de deux formes galéniques unitaires ou plus.

5. Composition pharmaceutique orale pour une utilisation selon la revendication 1 ou 2, dans laquelle R est -C₁₃H₂₅O et lorsqu'une dose quotidienne de 420 mg à 850 mg d'ester de testostérone est administrée de manière continue une fois par jour à chaque sujet dans un groupe de mâles hypogonadiques pendant une période d'au moins 84 jours, l'administration est telle qu'au moins 75 % des mâles hypogonadiques dans le groupe ont une C_{avg} de testostérone sérique d'environ 300 ng/dl à environ 1 100 ng/dl, et au moins l'une des suivantes :
une Cₘₐₓ de testostérone sérique de moins de 1 500 ng/dl chez au moins 85 % des sujets dans le groupe ;
une Cₘₐₓ de testostérone sérique d'environ 1 800 ng/dl à environ 2 500 ng/dl chez 10 % ou moins des sujets dans le groupe ; et
une Cₘₐₓ de testostérone sérique supérieure à 2 500 ng/dl chez environ 5 % ou moins des sujets dans le groupe.

6. Composition pharmaceutique orale pour une utilisation selon la revendication 1 ou 2, dans laquelle R est -C₁₄H₂₇O et lorsqu'une dose quotidienne de 525 mg à 1 250 mg d'ester de testostérone est administrée de manière continue une fois par jour à chaque sujet dans un groupe de mâles hypogonadiques pendant une période d'au moins 84 jours, l'administration est telle qu'au moins 75 % des mâles hypogonadiques dans le groupe ont une C_{avg} de testostérone sérique d'environ 300 ng/dl à environ 1 100 ng/dl, et au moins l'une des suivantes :
une Cₘₐₓ de testostérone sérique de moins de 1 500 ng/dl chez au moins 85 % des sujets dans le groupe ;
une Cₘₐₓ de testostérone sérique d'environ 1 800 ng/dl à environ 2 500 ng/dl chez 10 % ou moins des sujets dans le groupe ; et
une Cₘₐₓ de testostérone sérique supérieure à 2 500 ng/dl chez environ 5 % ou moins des sujets dans le groupe.

7. Composition pharmaceutique orale pour une utilisation selon la revendication 1 ou 2, dans laquelle le vecteur pharmaceutiquement acceptable inclut un additif lipophile qui est un tensioactif lipophile.
